# EUROPEAN PATENT APPLICATION

(11) **EP 4 361 140 A1**
(43) Date of publication of application: **01.05.2024**
(21) Application number: 22810666.2
(22) Date of filing: 27.05.2022
(51) Int. Cl.: C07D 401/14, A61K 31/4439, A61P 31/18

(54) **PHARMACEUTICALLY ACCEPTABLE SALT AND CRYSTAL FORM OF FUSED PYRIDINE RING DERIVATIVE AND PREPARATION METHOD THEREFOR**

(30) Priority: 28.05.2021 CN 202110589463
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: FENG, Yingqiang, Shanghai 200245 (CN); XI, Zhuoxun, Shanghai 200245 (CN); FENG, Jun, Shanghai 200245 (CN); HE, Feng, Shanghai 200245 (CN); WU, Qi, Lianyungang, Jiangsu 222047 (CN); DU, Zhenxing, Lianyungang, Jiangsu 222047 (CN)
(74) Representative: Potter Clarkson
(86) International application number: PCT/CN2022/095748
(87) International publication number: WO 2022/247942

(57) **Abstract**

The present disclosure relates to a pharmaceutically acceptable salt and a crystal form of a fused pyridine ring derivative and a preparation method therefor. Specifically, the present disclosure relates to a choline salt, sodium salt, potassium salt, calcium salt, arginine salt, lysine salt, meglumine salt, ethanolamine salt, hydrosulfate, hydrochloride, tartrate, maleate, citrate, malate, p-toluenesulfonate, and mesylate, and a crystalline form of a compound as represented by formula (I).

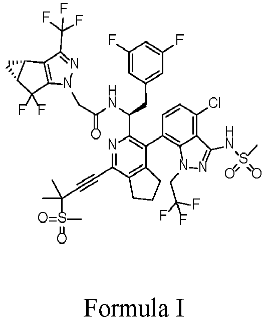

## Description

This application claims the priority of Chinese patent application 2021105894636, filed on May 28, 2021. The content of the above Chinese patent application is incorporated herein by reference in its entirety.

### TECHNICAL FIELD

The present disclosure relates to pharmaceutically acceptable salts and crystal forms of a fused pyridine ring derivative and preparation method therefor. Specifically, it provides a choline salt, sodium salt, potassium salt, calcium salt, arginine salt, lysine salt, meglumine salt, ethanolamine salt, hydrosulfate, hydrochloride, tartrate, maleate, citrate, malate, p-toluenesulfonate, and mesylate, and crystal forms of a compound of formula (I).

### BACKGROUND

AIDS (acquired immunodeficiency syndrome), which is a fatal infectious disease caused by human immunodeficiency virus (HIV), has the pathogenesis that the functions of CD4⁺T lymphocytes are damaged and largely destroyed under the direct and indirect action of the HIV virus, causing cellular immunodeficiency and various serious opportunistic infections and tumorigenesis. Today, over 35 million of people worldwide have been infected with the HIV virus.

Current therapy for HIV infected patients is composed of combinations of highly active antiretroviral drugs (HAART) mainly including nucleotide reverse transcriptase inhibitors (NRTIs), non-nucleotide reverse transcriptase inhibitors (NNRTIs), protease inhibitors (PIs), integrase strand transfer inhibitors (INIs) or entry inhibitors. These drugs effectively inhibit viral load and rate of transmission by targeting viral enzymes or viral proteins at various stages in the HIV viral replication cycle, thus significantly delaying the progression of the disease, thereby prolonging the life of the patient (Engelman, A., Cherepanov, P., Nature Reviews 2012, 10, 279-290; Flexner, C., Nature Rev Drug Discov. 2007, 6, 959-966).

However, these combination therapies are susceptible to the development of drug-resistant strains of HIV due to the rapid replication and high mutation rate of human immunodeficiency virus type 1 (HIV-1), which ultimately leads to drug failure, viral escape and exacerbation (Grant, R. M., Hecht, F. M., Warmerdam, M., JAMA 2002, 288, 181-188-559; Smith, R. J., Okano, J. T., Kahn, J. S., Bodine, E. N., Blower, S., Science 2010, 327, 697-701). Therefore, there is an urgent need to develop novel antiretroviral drugs that are active against the newly emerging drug-resistant HIV variants. In particular, novel developed drugs demonstrate high genetic disorder on drug resistance and have higher safety and patient compliance than the existing drugs.

WO2021104413 provides a compound of formula I, and the chemical name is *N*-((*S*)-1-((*R*)-4-(4-chloro-3-(methanesulfonamide)-1-(2,2,2-trifluoroethyl)-1*H*-indazol-7-yl)-1-(3-methyl-3-(methylsulfonyl)but-1-yn-1-yl)-6,7-dihydro-5*H*-cyclopenta[c]pyridin-3-yl)-2-(3,5-difluorophenyl)ethyl)-2-((3b*S*,4a*R*)-5,5-difluoro-3-(trifluoromethyl)-3b,4, 4a,5-tetrahydro-1*H-*cyclopropa[3,4]cyclopenta[1,2-c]pyrazol-1-yl)acetamide, and this compound has a good inhibitory effect on HIV-1 capsid protein,

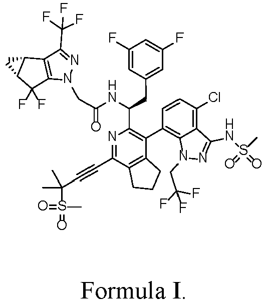

### CONTENT OF THE PRESENT INVENTION

The present disclosure provides a pharmaceutically acceptable salt of a compound of formula (I), wherein the pharmaceutically acceptable salt is selected from choline salt, sodium salt, potassium salt, calcium salt, arginine salt, lysine salt, meglumine salt, ethanolamine salt, hydrosulfate, hydrochloride, tartrate, maleate, citrate, malate, p-toluenesulfonate and mesylate. The chemical name of the compound of formula I is *N*-((*S*)-1-((*R*)-4-(4-chloro-3-(methanesulfonamide)-1-(2,2,2-trifluoroethyl)-1*H*-indazol-7-yl)-1-(3-methyl-3-(methylsulfonyl)but-1-yn-1-yl)-6,7-dihydro-5*H*-cyclopenta[c]pyridin-3-yl)-2-(3,5-difluorophenyl)ethyl)-2-((3bS,4aR)-5,5-difluoro-3-(trifluoromethyl)-3b,4, 4a,5-tetrahydro-1*H-*cyclopropa[3,4]cyclopenta[1,2-c]pyrazol-1-yl) acetamide,

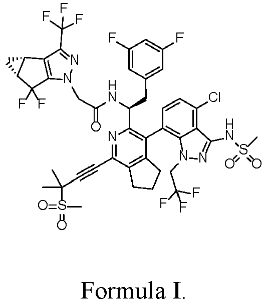

In some embodiments, the chemical ratio of the compound of formula (I) to the base molecules or acid molecules is 1:0.5 to 1:3, preferably 1:0.5, 1:1, 1:2 or 1:3, and most preferably 1:1 or 1:2. In some embodiments, the chemical ratio of the compound of formula (I) to sodium ions is 1:1. In some embodiments, the chemical ratio of the compound of formula (I) to choline is 1:1. In some embodiments, the chemical ratio of the compound of formula (I) to potassium ions is 1:1. In some embodiments, the chemical ratio of the compound of formula (I) to hydrogen sulfate is 1:1.

The present disclosure provides a method for preparing a pharmaceutically acceptable salt of a compound of formula (I), comprising the step of forming a salt of the compound of formula (1) with a base or acid. In some embodiments, the solvent used for the salt formation reaction is selected from one or more of isopropanol, methyl tert-butyl ether, isopropyl ether, ethanol, ethyl acetate, acetonitrile, dichloromethane, water, acetone and n-heptane.

In some embodiments, the method for preparing the foregoing pharmaceutically acceptable salt further comprises steps of volatilizing the solvent or stirring for crystallization, filtering, drying, etc.

The present disclosure provides a pharmaceutical composition prepared from the foregoing pharmaceutically acceptable salt.

The present disclosure provides a pharmaceutical composition comprising the foregoing pharmaceutically acceptable salt or the pharmaceutically acceptable salt prepared by the foregoing method, and optionally a pharmaceutically acceptable carrier, diluent or excipient.

The present disclosure provides a method for preparing a pharmaceutical composition, comprising a step of mixing the foregoing pharmaceutically acceptable salt or the pharmaceutically acceptable salt of the compound of formula (I) prepared by the foregoing method, and a pharmaceutically acceptable carrier, diluent or excipient.

The present disclosure provides a use of the foregoing pharmaceutically acceptable salt of the compound of formula (I), or a pharmaceutically acceptable salt prepared by the foregoing method, or the foregoing composition, or the composition prepared by the foregoing method in the preparation of HIV capsid protein inhibitors.

The present disclosure provides a use of the foregoing pharmaceutically acceptable salt of the compound of formula (I), or a pharmaceutically acceptable salt prepared by the foregoing method, or the foregoing composition, or the composition prepared by the foregoing method in the manufacture of a medicament for the prevention and/or treatment of a viral infection disease, preferably HIV infection.

The present disclosure provides an amorphous form of the choline salt of the compound of formula (I), wherein the X-ray powder diffraction pattern has no significant characteristic diffraction peaks at the 2θ angles of 2 to 48°.

The present disclosure further provides a method for preparing the amorphous form of the choline salt of the compound of formula (I), selected from: method 1, a) mixing the choline salt of the compound of formula (I) with isopropanol and choline hydroxide solution, b) adding dichloromethane, dissolving, and concentrating;
or method 2, a) mixing the compound of formula (I) with methyl tert-butyl ether and choline hydroxide solution, b) adding n-heptane and the solid precipitates.

In some embodiments, the volume(µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of choline salt as described in the present disclosure further comprises steps of filtering, washing, or drying.

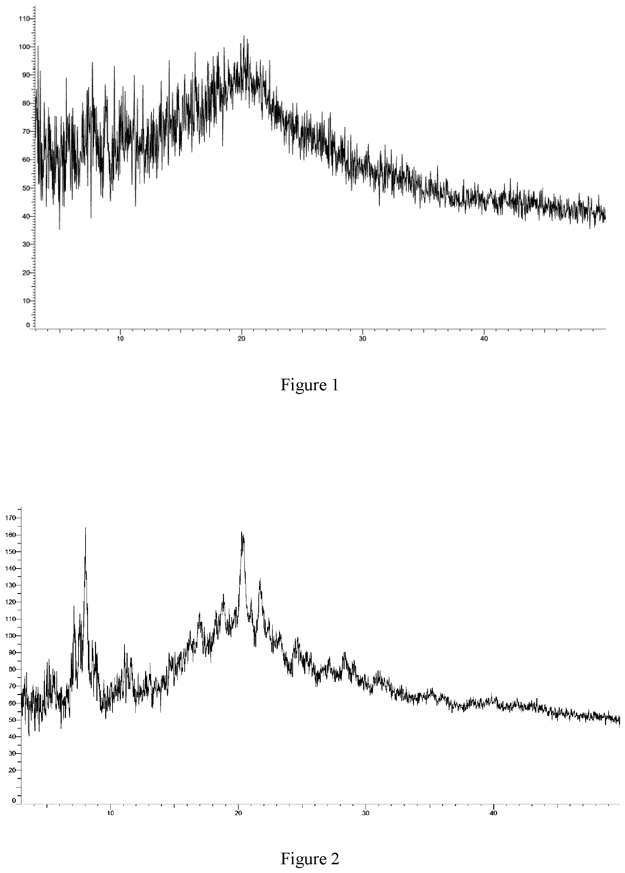

The present disclosure provides a crystal form A of choline salt of a compound of formula (I), which has an X-ray powder diffraction pattern comprising characteristic peaks at 2θ angles of 5.183, 8.040, 11.116, 16.998, 18.845, 20.322, and 21.726. In some embodiments, the X-ray powder diffraction pattern expressed by the diffraction angle 2θ of the crystal form A of the choline salt of the compound of formula (I) is as shown in Figure 2.

The present disclosure further provides a method for preparing the crystal form A of the choline salt of the compound of formula (I), selected from: method 1, a) mixing the amorphous form of the choline salt of the compound of formula (I) with solvent I, wherein the solvent I is selected from one or more of ethyl acetate, n-heptane and isopropyl acetate, b) pulping and crystallizing;
or method 2, a) mixing the amorphous form of the choline salt of the compound of formula (I) with a mixture of dioxane/n-hexane/isopropyl ether, or 4-methyl-2-pentanone, and b) stirring and crystallizing.

In some embodiments, the volume of solvent used in the present disclosure (µl) can be 1-200 times the mass (mg) of the compound of formula I, and in non-limting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the A crystal form of the choline salt as described in the present disclosure further comprises steps of filtering, washing, or drying, etc.

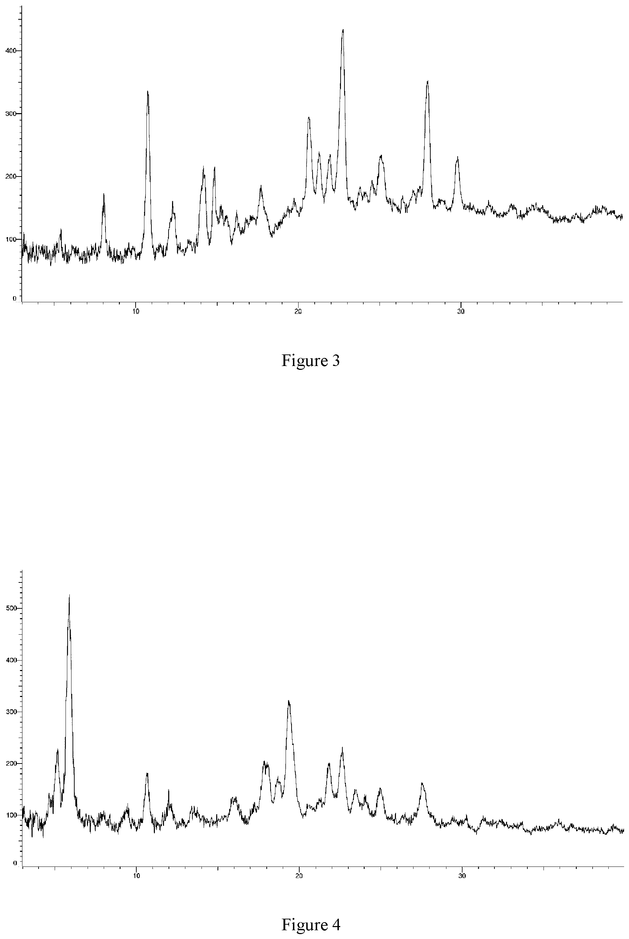

The present disclosure provides a crystal form B of choline salt of a compound of formula (I), which has an X-ray powder diffraction pattern comprising characteristic peaks at 2θ angles of 8.056, 10.761, 14.170, 14.835, 20.657, 22.751, and 27.938. In some embodiments, the crystal form B of the choline salt of the compound of formula (I) has characteristic peaks at 8.056, 10.761, 12.289, 14.170, 14.835, 20.657, 21.279, 21.944, 22.751 and 27.938. In some embodiments, the crystal form B of the choline salt of the compound of formula (I) has characteristic peaks at 8.056, 10.761, 12.289, 14.170, 14.835, 17.676, 20.657, 21.279, 21.944, 22.751, 25.061, 27.938 and 29.791. In some embodiments, the X-ray powder diffraction pattern expressed by the diffraction angle 2θ of the crystal form B of the choline salt of the compound of formula (I) is as shown in Figure 3.

The present disclosure further provides a method for preparing the crystal form B of the choline salt of the compound of formula (I), selected from:
method 1, a) mixing the compound of formula I with choline hydroxide and solvent II, wherein the solvent II is selected from at least one of ethanol and isopropanol, b) pulping and crystallizing
or method 2, a) mixing the compound of formula (I) with choline hydroxide and ethanol, b) adding isopropanol or n-heptane and crystallizing;
or method 3, mixing the amorphous form of the choline salt of the compound of formula I with solvent III, wherein the solvent III is selected from one or more of methanol, isopropyl ether, acetonitrile, isopropanol, ethanol, dichloromethane, water and n-hexane, b) pulping and crystallizing;
or method 4, a) mixing crystal form A of the choline salt of the compound of formula I with at least one of isopropanol, methylene chloride and n-hexane, and b) pulping and crystallizing.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form B of the choline salt as described in the present disclosure further comprises steps of filtering, washing, or drying, etc.

The present disclosure provides an amorphous form of the sodium salt of the compound of formula (I), wherein the X-ray powder diffraction pattern has no significant characteristic diffraction peaks at the 2θ angles of 2 to 48°.

The present disclosure further provides a method for preparing the amorphous form of the sodium salt of the compound of formula (I), comprising the steps: method a) mixing the compound of formula (I) with at least one solvent selected from ethyl acetate, acetonitrile and dichloromethane, and sodium hydroxide solution; b) adding n-heptane to precipitate the solid.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of sodium salt as described in the present disclosure further comprises steps of filtering, washing, or drying.

The present disclosure provides a crystal form I of sodium salt of a compound of formula (I), which has an X-ray powder diffraction pattern comprising characteristic peaks at 2θ angles of 5.167, 5.869, 10.671, 17.850, 19.388, 22.646, and 27.604. In some embodiments, the crystal form I of the sodium salt of the compound of formula (I) has characteristic peaks at 5.167, 5.869, 10.671, 16.031, 17.850, 18.677, 19.388, 22.646, 24.974 and 27.604. In some embodiments, the crystal form I of the sodium salt of the compound of formula (I) has characteristic peaks at 5.167, 5.869, 9.409, 10.671, 12.035, 16.031, 17.850, 18.677, 19.388, 21.811, 22.646, 23.483, 24.974 and 27.604. In some embodiments, the X-ray powder diffraction pattern expressed by the diffraction angle 2θ of the crystal form I of the sodium salt of the compound of formula (I) is as shown in Figure 4.

The present disclosure further provides a method for preparing the crystal form I of the sodium salt of the compound of formula (I), selected from: a) mixing the compound of formula (I) with methyl tert-butyl ether and choline hydroxide solution, and heating, b) stirring and crystallizing.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form I of the sodium salt as described in the present disclosure further comprises steps of filtering, washing, or drying.

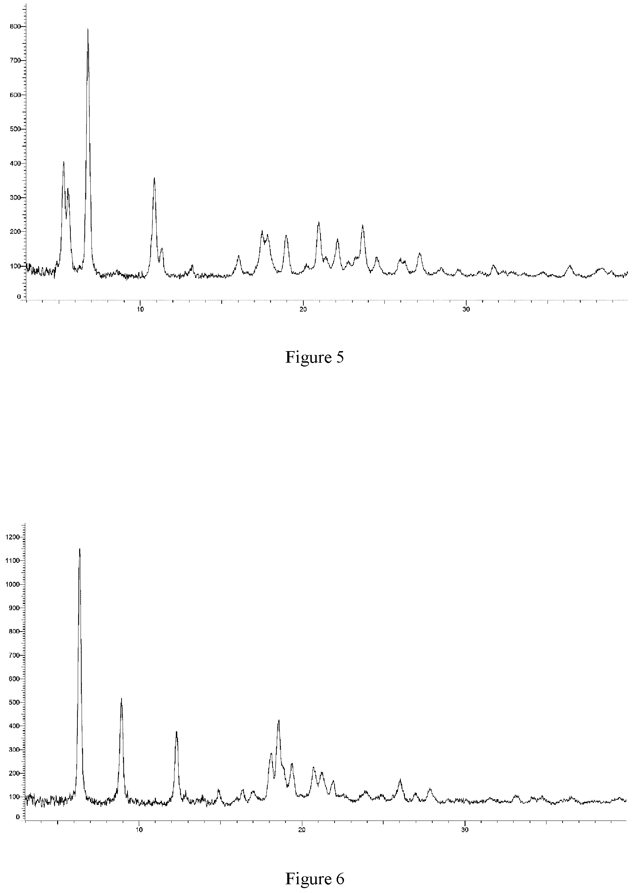

The present disclosure provides a crystal form II of the sodium salt of the compound of formula (I), which has an X-ray powder diffraction pattern comprising characteristic peaks at 2θ angles of 5.293, 6.788, 10.864, 17.477, 18.966, 20.950, and 23.653. In some embodiments, the crystal form II of the sodium salt of the compound of formula (I) has characteristic peaks at 5.293, 6.788, 10.864, 16.027, 17.477, 18.966, 20.950, 22.115, 23.653 and 27.156. In some embodiments, the crystal form II of the sodium salt of the compound of formula (I) has characteristic peaks at 5.293, 5.561, 6.788, 10.864, 11.332, 13.181, 16.027, 17.477, 18.966, 20.950, 22.115, 23.653, 24.509, 25.979 and 27.156. In some embodiments, the X-ray powder diffraction pattern expressed by the diffraction angle 2θ of the crystal form II of the sodium salt of the compound of formula (I) is as shown in Figure 5.

The present disclosure further provides a method for preparing the crystal form II of the sodium salt of the compound of formula (I), selected from: method 1, a) mixing the compound of formula I with methyl tert-butyl ether and sodium hydroxide solution, b) stirring and crystallizing;
or method 2, a) heating the crystal form I of the sodium salt of the compound of formula Ito 160°C;
or method 3, a) mixing the compound of formula I with isopropanol and sodium hydroxide solution, b) adding n-heptane, pulping and crystallizing.

In some embodiments, the volume (µl) of solvent used in the present disclosure can be 1-200 times the mass (mg) of the compound of formula (I), and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form II of the sodium salt as described in the present disclosure further comprises steps of filtering, washing, or drying.

The present disclosure provides a crystal form III of sodium salt of a compound of formula (I), which has an X-ray powder diffraction pattern comprising characteristic peaks at 2θ angles of 6.352, 8.913, 12.297, 18.141, 18.585, 19.373, and 20.718. In some embodiments, the crystal form III of the sodium salt of the compound of formula (I) has characteristic peaks at 6.352, 8.913, 12.297, 18.141, 18.585, 19.373, 20.718, 21.216, 21.938 and 26.014. In some embodiments, the crystal form III of the sodium salt of the compound of formula (I) has characteristic peaks at 6.352, 8.913, 12.297, 14.919, 16.346, 18.141, 18.585, 19.373, 20.718, 21.216, 21.938, 26.014 and 27.864. In some embodiments, the X-ray powder diffraction pattern expressed by the diffraction angle 2θ of the crystal form III of the sodium salt of the compound of formula (I) is as shown in Figure 6.

The present disclosure further provides a method for preparing the crystal form III of the sodium salt of the compound of formula (I), comprising steps: method a) mixing the compound of formula I with isopropanol and sodium hydroxide solution, b) stirring and crystallizing;

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form III of sodium salt as described in the present disclosure further comprises steps of filtering, washing, or drying, etc.

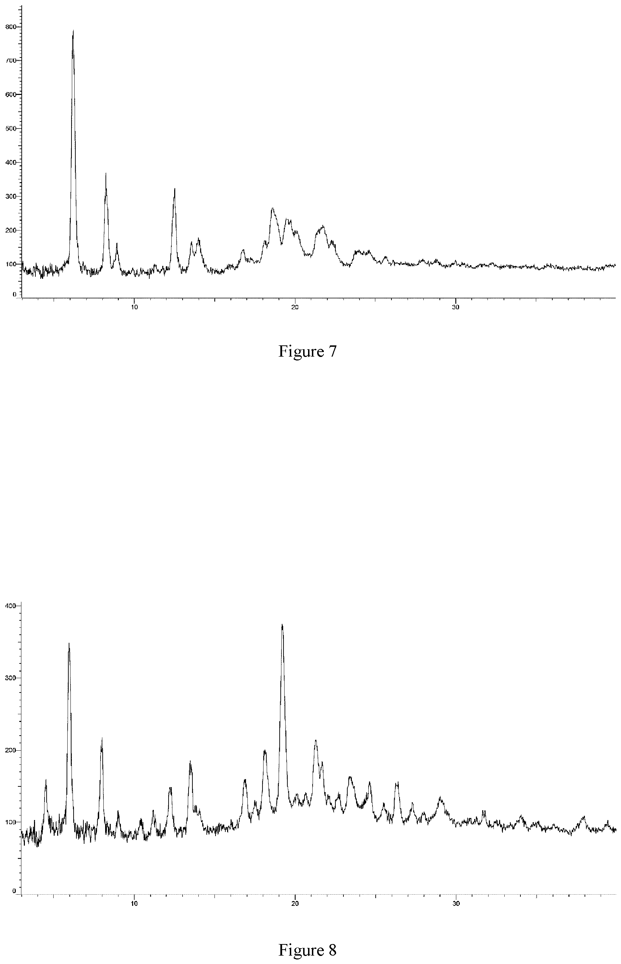

The present disclosure provides a crystal form IV of sodium salt of a compound of formula (I), which has an X-ray powder diffraction pattern comprising characteristic peaks at 2θ angles of 6.179, 8.242, 12.509, 13.986, 18.625, 19.522, and 21.746. In some embodiments, the crystal form IV of the sodium salt of the compound of formula (I) has characteristic peaks at 6.179, 8.242, 8.914, 12.509, 13.986, 18.625, 19.522, 21.454, 21.746 and 22.280. In some embodiments, the crystal form IV of the sodium salt of the compound of formula (I) has characteristic peaks at 6.179, 8.242, 8.914, 12.509, 13.986, 16.762, 18.625, 19.522, 21.454, 21.746, 22.280 and 23.836. In some embodiments, the X-ray powder diffraction pattern expressed by the diffraction angle 2θ of the IV crystal form of the sodium salt of the compound of formula (I) is as shown in Figure 7.

The present disclosure further provides a method for preparing the crystal form IV of the sodium salt of the compound of formula (I), comprising steps: a) mixing the compound of formula I with ethanol and sodium hydroxide solution, b) stirring and crystallizing.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form IV of sodium salt as described in the present disclosure further comprises steps of filtering, washing, or drying, etc.

The present disclosure provides a crystal form V of sodium salt of a compound of formula (I), which has an X-ray powder diffraction pattern comprising characteristic peaks at 2θ angles of 4.506, 5.957, 7.986, 13.490, 18.147, 19.213, and 21.300. In some embodiments, the crystal form V of the sodium salt of the compound of formula (I) has characteristic peaks at 4.506, 5.957, 7.986, 12.219, 13.490, 16.883, 18.147, 19.213, 21.300 and 26.323. In some embodiments, the crystal form V of the sodium salt of the compound of formula (I) has characteristic peaks at 4.506, 5.957, 7.986, 11.172, 12.219, 13.490, 16.883, 18.147, 19.213, 21.300, 23.434, 24.636 and 26.323. In some embodiments, the X-ray powder diffraction pattern expressed by the diffraction angle 2θ of the crystal form V of the sodium salt of the compound of formula (I) is as shown in Figure 8.

The present disclosure further provides a method for preparing the crystal form V of the sodium salt of the compound of formula (I), comprising steps: method 1, a) mixing the compound of formula (I) with isopropanol and sodium hydroxide solution, b) stirring and crystallizing;
or method 2, a) selecting one or more of the crystal form II, crystal form III and crystal form IV of the compound of formula I, and mixing it with isopropyl ether or isopropanol, b) pulping and crystallizing.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form V of sodium salt as described in the present disclosure further comprises steps of filtering, washing, or drying, etc.

The present disclosure provides an amorphous form of potassium salt of a compound of formula (I), wherein the X-ray powder diffraction pattern has no significant characteristic diffraction peaks at the 2θ angles of 2 to 48°.

The present disclosure further provides a method for preparing the amorphous form of the potassium salt of the compound of formula (I), comprising the steps: a) mixing the compound of formula (I) with acetone and potassium hydroxide solution, b) volatilizing and the solid precipitates.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of potassium salt as described in the present disclosure further comprises steps of filtering, washing, or drying.

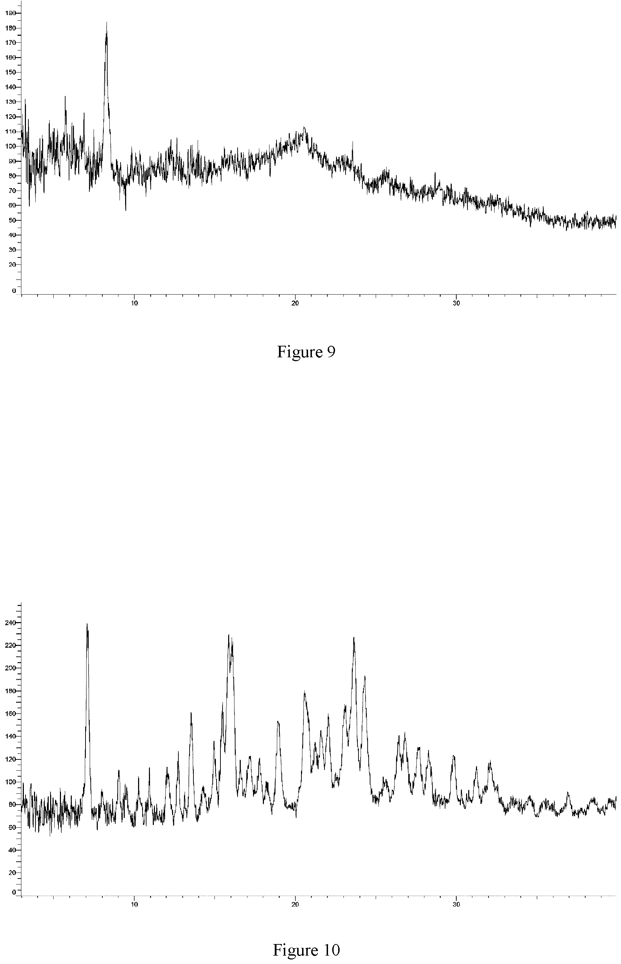

The present disclosure provides a crystal form a of potassium salt of a compound of formula (I), which has an X-ray powder diffraction pattern comprising characteristic peaks at 2θ angles of 5.746, 8.304, 12.253 and 20.503. In some embodiments, the X-ray powder diffraction pattern expressed by the diffraction angle 2θ of the crystal form a of the potassium salt of the compound of formula (I) is as shown in Figure 9.

The present disclosure further provides a method for preparing the crystal form a of the potassium salt of the compound of formula (I), comprising steps: a) mixing the compound of formula (I) with methyl tert-butyl ether and potassium hydroxide solution, b) stirring and crystallizing.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form a of the potassium salt as described in the present disclosure further comprises steps of filtering, washing, or drying, etc.

The present disclosure provides an amorphous form of hydrosulfate of a compound of formula (I), wherein the X-ray powder diffraction pattern has no significant characteristic diffraction peaks at the 2θ angles of 2 to 48°.

The present disclosure further provides a method for preparing the amorphous form of the hydrosulfate of the compound of formula (I), comprising the steps: a) mixing the compound of formula (I) with ethyl acetate and sulfuric acid aqueous solution, b) volatilizing and crystallizing.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of hydrosulfate as described in the present disclosure further comprises steps of filtering, washing, or drying.

The present disclosure provides a crystal form A of hydrosulfate of a compound of formula (I), which has an X-ray powder diffraction pattern comprising characteristic peaks at 2θ angles of 7.101, 13.538, 15.870, 18.959, 20.570, 23.649, and 24.320. In some embodiments, the crystal form A of the hydrosulfate of the compound of formula (I) has characteristic peaks at 7.101, 12.742, 13.538, 15.870, 18.959, 20.570, 21.603, 22.055, 23.649 and 24.320. In som2-e embodiments, the crystal form A of the hydrosulfate of the compound of formula (I) has characteristic peaks at 7.101, 10.933, 12.742, 13.538, 14.966, 15.870, 18.959, 20.570, 21.603, 22.055, 23.649, 24.320 and 26.799. In some embodiments, the X-ray powder diffraction pattern expressed by the diffraction angle 2θ of the crystal form A of the hydrosulfate of the compound of formula (I) is as shown in Figure 10.

The present disclosure further provides a method for preparing the crystal form A of the hydrosulfate of the compound of formula (I), comprising: a) mixing the compound of formula I with methyl tert-butyl ether, sulfuric acid solution or ethanol/water solution, b) stirring and crystallizing.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the crystal form A of hydrosulfate as described in the present disclosure further comprises steps of filtering, washing, or drying, etc.

The present disclosure provides an amorphous form of calcium salt of a compound of formula (I), wherein the X-ray powder diffraction pattern has no significant characteristic diffraction peaks at the 2θ angles of 2 to 48°.

The present disclosure further provides a method for preparing the amorphous form of the calcium salt of the compound of formula (I), comprising the steps: a) mixing the compound of formula (I) with acetone and calcium hydroxide solution, b) volatilizing and the solid precipitates.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of calcium salt as described in the present disclosure further comprises steps of filtering, washing, or drying.

The present disclosure provides an amorphous form of arginine salt of the compound of formula (I), wherein the X-ray powder diffraction pattern has no significant characteristic diffraction peaks at the 2θ angles of 2 to 48°.

The present disclosure further provides a method for preparing the amorphous form of the arginine salt of the compound of formula (I), comprising the steps: a) mixing the compound of formula (I) with ethanol/water and arginine solution, b) volatilizing and the solid precipitates.

In some embodiments, the volume(µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of arginine salt as described in the present disclosure further comprises steps of filtering, washing, or drying.

The present disclosure provides an amorphous form of lysine salt of a compound of formula (I), wherein the X-ray powder diffraction pattern has no significant characteristic diffraction peaks at the 2θ angles of 2 to 48°.

The present disclosure further provides a method for preparing the amorphous form of the lysine salt of the compound of formula (I), comprising the steps: a) mixing the compound of formula (I) with methyl tert-butyl ether and lysine solution, b) volatilizing and the solid precipitates.

In some embodiments, the volume(µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of lysine salt as described in the present disclosure further comprises steps of filtering, washing, or drying.

The present disclosure provides an amorphous form of meglumine salt of the compound of formula (I), wherein the X-ray powder diffraction pattern has no significant characteristic diffraction peaks at the 2θ angles of 2 to 48°.

The present disclosure further provides a method for preparing the amorphous form of the compound amorphous meglumine salt of formula (I), comprising the steps: a) mixing the compound of formula (I) with acetone and meglumine solution, b) volatilizing and the solid precipitates.

In some embodiments, the volume(µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of meglumine salt as described in the present disclosure further comprises steps of filtering, washing, or drying.

The present disclosure provides an amorphous form of ethanolamine salt of a compound of formula (I), wherein the X-ray powder diffraction pattern has no significant characteristic diffraction peaks at the 2θ angles of 2 to 48°.

The present disclosure further provides a method for preparing the amorphous form of the ethanolamine salt of the compound of formula (I), comprising the steps: a) mixing the compound of formula (I) with ethanol/water, ethanolamine solution, b) volatilizing and the solid precipitates.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of the ethanolamine salt as described in the present disclosure further comprises steps of filtering, washing, or drying.

The present disclosure provides an amorphous form of hydrochloride of a compound of formula (I), wherein the X-ray powder diffraction pattern has no significant characteristic diffraction peaks at the 2θ angles of 2 to 48°.

The present disclosure further provides a method for preparing the amorphous form of the hydrochloride of the compound of formula (I), comprising the steps: a) mixing the compound of formula (I) with ethanol/water, hydrochloride solution, b) volatilizing and the solid precipitates.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of hydrochloride as described in the present disclosure further comprises steps of filtering, washing, or drying.

The present disclosure provides an amorphous form of tartrate of a compound of formula (I), wherein the X-ray powder diffraction pattern has no significant characteristic diffraction peaks at the 2θ angles of 2 to 48°.

The present disclosure further provides a method for preparing the amorphous form of the tartrate of the compound of formula (I), comprising the steps: a) mixing the compound of formula (I) with ethanol/water, tartrate solution, b) volatilizing and the solid precipitates.

In some embodiments, the volume(µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of the tartrate as described in the present disclosure further comprises steps of filtering, washing, or drying.

The present disclosure provides an amorphous form of maleate of a compound of formula (I), wherein the X-ray powder diffraction pattern has no significant characteristic diffraction peaks at the 2θ angles of 2 to 48°.

The present disclosure further provides a method for preparing the amorphous form of the maleate of the compound of formula (I), comprising the steps: a) mixing the compound of formula (I) with methyl tert-butyl ether and lysine solution, b) volatilizing and the solid precipitates.

In some embodiments, the volume(µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of maleate as described in the present disclosure further comprises steps of filtering, washing, or drying.

The present disclosure provides an amorphous form of citrate of a compound of formula (I), wherein the X-ray powder diffraction pattern has no significant characteristic diffraction peaks at the 2θ angles of 2 to 48°.

The present disclosure further provides a method for preparing the amorphous form of the citrate of the compound of formula (I), comprising the steps: a) mixing the compound of formula (I) with methyl tert-butyl ether and lysine solution, b) volatilizing and the solid precipitates.

In some embodiments, the volume (µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of citrate as described in the present disclosure further comprises steps of filtering, washing, or drying.

The present disclosure provides an amorphous form of malate of a compound of formula (I), wherein the X-ray powder diffraction pattern has no significant characteristic diffraction peaks at the 2θ angles of 2 to 48°.

The present disclosure further provides a method for preparing the amorphous form of the malate of the compound of formula (I), comprising the steps: a) mixing the compound of formula (I) with methyl tert-butyl ether and malic acid solution, b) volatilizing and the solid precipitates.

In some embodiments, the volume(µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of malate as described in the present disclosure further comprises steps of filtering, washing, or drying.

The present disclosure provides an amorphous form of the p-toluenesulfonate of the compound of formula (I), wherein the X-ray powder diffraction pattern has no significant characteristic diffraction peaks at the 2θ angles of 2 to 48°.

The present disclosure further provides a method for preparing the amorphous form of the p-toluenesulfonate of the compound of formula (I), comprising the steps: a) mixing the compound of formula (I) with methyl tert-butyl ether and p-toluenesulfonate solution, b) volatilizing and the solid precipitates.

In some embodiments, the volume(µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of p-toluenesulfonate as described in the present disclosure further comprises steps of filtering, washing, or drying.

The present disclosure provides an amorphous form of mesylate of a compound of formula (I), wherein the X-ray powder diffraction pattern has no significant characteristic diffraction peaks at the 2θ angles of 2 to 48°.

The present disclosure further provides a method for preparing the amorphous form of the mesylate of the compound of formula (I), including the steps: a) mixing the compound of formula (I) with methyl tert-butyl ether and mesylate solution, b) volatilizing and the solid precipitates.

In some embodiments, the volume(µl) of solvent used in the present disclosure can be 1-200 times the mass (mg) of the compound of formula I, and in non-limiting embodiments, it can be 1, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 200. In some embodiments, the method for preparing the amorphous form of mesylate as described in the present disclosure further comprises steps of filtering, washing, or drying.

The present disclosure provides a pharmaceutical composition prepared from the crystal form of the pharmaceutically acceptable salt of the compound represented by the foregoing formula (I).

The present disclosure further provides a pharmaceutical composition comprising the crystal form of the foregoing pharmaceutically acceptable salt and optionally a pharmaceutically acceptable carrier, diluent or excipient.

The present disclosure further provides a method for preparing a pharmaceutical composition, comprising the step of mixing the crystal form of the foregoing pharmaceutically acceptable salt with a pharmaceutically acceptable carrier, diluent or excipient.

The present disclosure further provides a use of the crystal form of the foregoing pharmaceutically acceptable salt, or the foregoing composition, or the composition prepared by the foregoing method in the preparation of HIV capsid protein inhibitors.

The present disclosure further provides a use of the crystal form of the foregoing pharmaceutically acceptable salt, or the foregoing composition, or the composition prepared by the foregoing method in the manufacture of a medicament for preventing and/or treating viral infection diseases, preferably HIV infection.

There is a certain degree of error in the determination of the chemical ratio of the compound of formula (I) and the base molecules or acid molecules in the present disclosure. Generally speaking, plus or minus 10% are within a reasonable range of error. There is a certain degree of error variation with the context where it is used, and the error variation does not exceed plus or minus 10%, plus or minus 9%, plus or minus 8%, plus or minus 7%, plus or minus 6%, plus or minus 5%, plus or minus 4%, plus or minus 3%, plus or minus 2%, plus or minus 1%, preferably plus or minus 5%.

The "2θ or 2θ angle" mentioned in the present disclosure refers to the diffraction angle, θ is the Bragg angle, and the unit is ° or degree; the error range of each characteristic peak 2θ is ±0.20 (including the rounding of numbers with more than one decimal place), and can be -0.20, -0.19, -0.18, -0.17, -0.16, -0.15, -0.14, -0.13, -0.12, -0.11, -0.10, -0.09, -0.08, -0.07, -0.06, -0.05, -0.04, -0.03, -0.02, -0.01, 0.00, 0.01, 0.02, 0.03, 0.04, 0.05, 0.06, 0.07, 0.08, 0.09, 0.10, 0.11, 0.12, 0.13, 0.14, 0.15, 0.16, 0.17, 0.18, 0.19, 0.20.

According to the description of hygroscopic characteristics and the definition of hygroscopic weight gain in the "9103 Guiding principles for drug hygroscopicity" in "Chinese Pharmacopoeia", Volume IV, 2015.
Deliquescence: Absorbing enough water to form a liquid;
Extremely hygroscopic: the hygroscopic weight gain is not less than 15%;
Possess hygroscopicity: the hygroscopic weight gain is less than 15% but not less than 2%;
Slightly hygroscopic: the hygroscopic weight gain is less than 2% but not less than 0.2%;
No or almost no hygroscopicity: the hygroscopicity gain is less than 0.2%.

"Differential scanning calorimetry or DSC" as described in the present disclosure refers to measuring the temperature difference and heat flow difference between the sample and the reference during the process of heating or constant temperature of the sample, in order to characterize all physical changes and chemical changes related to thermal effect to obtain phase change information of the sample.

The drying temperature described in the present disclosure is generally from 25°C to 150°C, preferably from 40°C to 80°C, and it can be dried at atmospheric pressure or at reduced pressure.

"Pharmaceutical composition" means a mixture containing one or more compound of formula (I) as described herein, or a pharmaceutically acceptable salt thereof and other chemical components, and other components such as pharmaceutically acceptable carriers and excipients. The purpose of pharmaceutical composition is to promote the administration to organisms, and facilitate the absorption of active ingredients to exert biological activity.

The crystal form described in the present disclosure include but are not limited to solvate of compound of formula (I). The solvents include but are not limited to ethyl acetate/n-heptane, isopropyl acetate/n-heptane, methanol/isopropyl ether, acetonitrile/isopropyl ether, dichloromethane/isopropyl ether, ethanol/water, water/isopropanol, ethanol/n-hexane, ethanol, isopropanol, dichloromethane/n-hexane, dioxane /n-hexane/isopropyl ether, 4-methyl-2-pentanone, methyl tert-butyl ether, ethanol, ethyl acetate, acetonitrile, dichloromethane, ethanol/isopropanol, ethanol/n-heptane.

"Solvate" as described in the present disclosure includes but is not limited to a complex formed by combining a compound of formula (I) with a solvent.

### BRIEF DESCRIPTION OF THE DRAWINGS

Figure 1: XRPD pattern of the amorphous form of the choline salt of the compound of formula (I).
Figure 2: XRPD pattern of the crystal form A of the choline salt of the compound of formula (I).
Figure 3: XRPD pattern of the crystal form B of the choline salt of the compound of formula (I).
Figure 4: XRPD pattern of the crystal form I of the sodium salt of the compound of formula (I).
Figure 5: XRPD pattern of the crystal form II of the sodium salt of the compound of formula (I).
Figure 6: XRPD pattern of the crystal form III of the sodium salt of the compound of formula (I).
Figure 7: XRPD pattern of the crystal form IV of the sodium salt of the compound of formula (I).
Figure 8: XRPD pattern of the crystal form V of the sodium salt of the compound of formula (I).
Figure 9: XRPD pattern of the crystal form a of the potassium salt of the compound of formula (I).
Figure 10: XRPD pattern of the crystal form A of the hydrosulfate of the compound of formula (I).

### DETAILED DESCRIPTION OF THE PREFERRED EMBODIMENT

The following examples or experimental examples further illustrate the present disclosure, but the present disclosure. The examples or experimental examples in the present disclosure are only used to illustrate the technical solutions in the present disclosure, and the substance and scope of the present disclosure is not limited.

The experimental methods for which no specific conditions are specified in the examples in this disclosure are usually in accordance with the conventional conditions or the conditions suggested by the manufacturers of raw materials or commodities. Reagents without specific source specified are commercially available conventional reagents.

The structure of the compound was determined by nuclear magnetic resonance (NMR) spectroscopy and/or mass spectrometry (MS). NMR shift (δ) is given in a unit of 10⁻⁶ (ppm). NMR spectra were measured using a Bruker AVANCE-400 nuclear magnetic resonance instrument, with deuterated dimethyl sulfoxide (DMSO-d₆), deuterated chloroform (CDCl₃) and deuterated methanol (CD₃OD) as determination solvents, with tetramethylsilane (TMS) as internal standard.

Mass spectra were measured using Agilent 1200/1290 DAD-6110/6120 Quadrupole MS liquid chromatography-mass spectrometry system (manufacturer: Agilent; MS model: 6110/6120 Quadrupole MS), waters ACQuity UPLC-QD/SQD (manufacturer: waters, MS model: waters ACQuity Qda Detector/waters SQ Detector) and THERMO Ultimate 3000-Q Exactive (manufacturer: THERMO, MS model: THERMO Q Exactive).

High performance liquid chromatography (HPLC) was performed using Agilent HPLC 1200DAD, Agilent HPLC 1200VWD and Waters HPLC e2695-2489 high pressure liquid chromatography.

Chiral HPLC was performed onAgilent 1260 DAD HPLC.

HPLC preparation was performed using Waters 2767, Waters 2767-SQ Detecor2, Shimadzu LC-20AP and Gilson-281 preparative chromatographs.

Chiral preparation was performed on a Shimadzu LC-20AP preparative chromatograph.

A CombiFlash Rf200 (TELEDYNE ISCO) system was used for rapid preparation.

Huanghai HSGF254 or Qingdao GF254 silica gel plates of specifications 0.15 mm to 0.2 mm were adopted for thin layer chromatography (TLC) analysis and 0.4 mm to 0.5 mm for TLC separation and purification.

The silica gel column chromatography generally used 200 to 300-mesh silica gel (Huanghai, Yantai) as the carrier.

The mean inhibition of kinase and the IC₅₀ value were measured using a NovoStar microplate reader (BMG, Germany).

Known starting materials disclosed herein may be synthesized using or according to methods known in the art, or may be purchased from ABCR GmbH & Co. KG, Acros Organics, Aldrich Chemical Company, Accela ChemBio Inc., Chembee Chemicals, and other companies.

In the examples, the reactions can be performed in an argon atmosphere or a nitrogen atmosphere unless otherwise specified.

An argon atmosphere or a nitrogen atmosphere means that the reaction flask is connected to a balloon containing about 1L of argon or nitrogen.

A hydrogen atmosphere means that the reaction flask is connected to a balloon containing about 1L of hydrogen.

Parr 3916EKX hydrogenator, Qinglan QL-500 hydrogenator or HC2-SS hydrogenator was used for pressurized hydrogenation reactions.

The hydrogenation reaction usually involved 3 cycles of vacuumization and hydrogen purge.

A CEM Discover-S 908860 microwave reactor was used for the microwave reaction.

In the examples, a solution refers to an aqueous solution unless otherwise specified.

There are no special instructions in the examples. The reaction temperature is room temperature, which is 20°C to 30°C.

The monitoring of the reaction progress in the examples was conducted by thin layer chromatography (TLC). The developing solvent for reactions, the eluent system for column chromatography purification and the developing solvent system for thin layer chromatography included: A: n-hexane/ethyl acetate system, and B: dichloromethane/methanol system. The volume ratio of the solvents was adjusted according to the polarity of the compound, or by adding a small amount of basic or acidic reagents such as triethylamine and acetic acid.

The testing conditions for the instruments used in the experiments in the present disclosure:
1. Differential Scanning Calorimeter (DSC)
   Instrument model: Mettler Toledo DSC 3+STARe System
   Purge gas: nitrogen; nitrogen purging speed: 50 mL/min
   Heating rate: 10.0 °C/min
   Temperature range: 25to 350°C (or 25°C to 300°C)
2. X-ray Powder Diffraction (XRPD)
   Instrument model: BRUKER D8 Discover X-ray powder diffractometer
   Ray: Monochrome Cu-KαRay (λ=1.5418Å)
   Scanning mode: θ/2θ, scanning range (2θrange): 3 to 50°
   Voltage: 40kV, current: 40mA
3. Thermogravimetric Analysis (TGA)
   Instrument model: Mettler Toledo TGA2
   Purge gas: nitrogen; nitrogen purge speed: 50 mL/min
   Heating rate: 10.0 °C/min
   Temperature range: 25 to 400°C (or 25°C to 350°C)
4. DVS is dynamic vapor sorption
   The test adopts SMS DVS Advantage, at 25 °C, with a humidity range of 0 to 95% and a step of 10%. The judgment standard is that the dM/dT of each gradient mass change is less than 0.002%, TMAX is 360 minutes, and the cycle is two times.
5. Cation chromatography
   Instrument model: DIONEX AQUION ion chromatograph, USA
   Detection method: conductivity; separation column: IonPac CS16-CG16
   Eluent: MSA 20Mm
   Flow rate: 1.0ml/min
6. Anion chromatography
   Instrument model: DIONEX INTEGRION HPIC ion chromatograph, USA
   Detection method: conductivity; separation column: DionexIonPac^{™}-AS11-HC
   Eluent: EGC-500-KOH
   Flow rate: 1.5ml/min

### Example 1: Preparation of compound of formula (I)

*N*-((*S*)-1-((*R*)-4-(4-chloro-3-(methanesulfonamide)-1-(2,2,2-trifluoroethyl)-1*H-*indazol-7-yl)-1-(3-methyl-3-(methylsulfonyl)but-1-yn-1-yl)-6,7-dihydro-5*H-*cyclopenta[c]pyridin-3-yl)-2-(3,5-difluorophenyl)ethyl)-2-((3b*S*,4a*R*)-5,5-difluoro-3-(trifluoromethyl)-3b,4,4a,5-tetrahydro-1*H*-cyclopropa[3,4]cyclopenta[1,2-c]pyrazol-1-yl)acetamide **I**

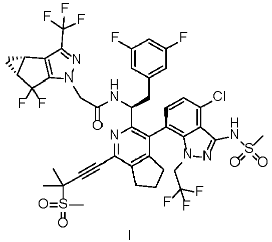

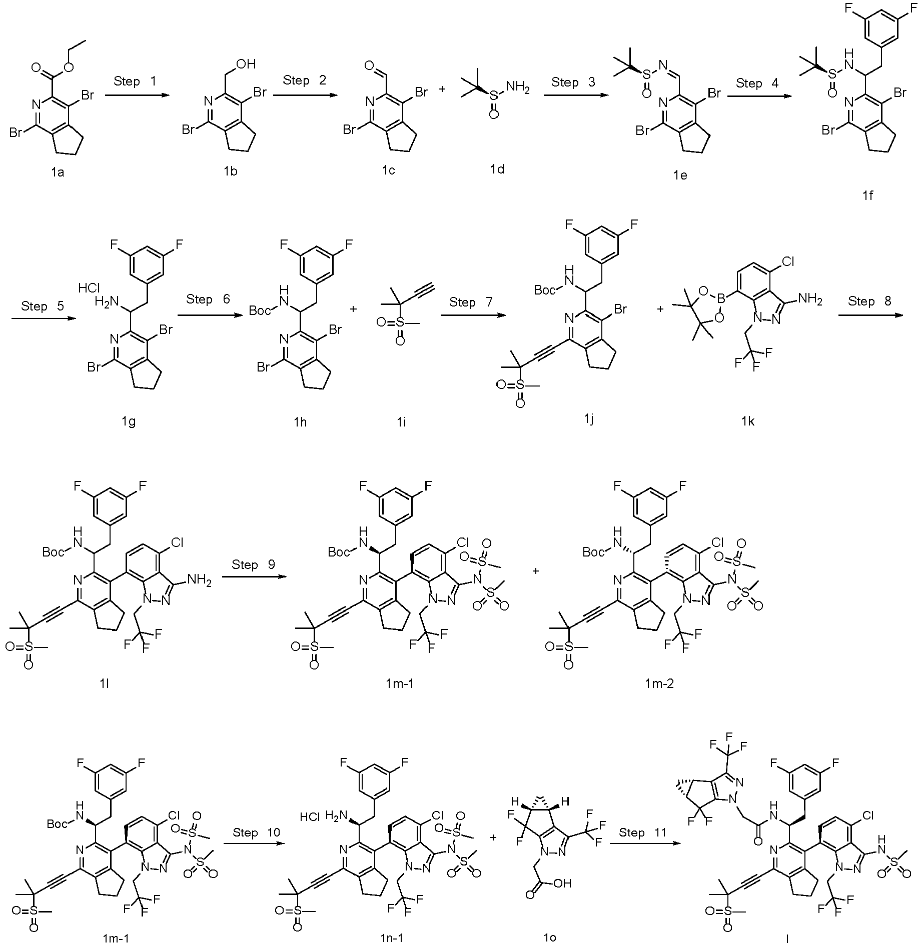

### Step 1

### (1,4-dibromo-6,7-dihydro-5H-cyclopenta[c]pyridin-3-yl)methanol 1b

Ethyl 1,4-dibromo-6,7-dihydro-5*H*-cyclopenta[c]pyridine-3-carboxylate **1a** (7.2 g, 20.6 mmol, prepared by a method known in the literature "Advanced Synthesis & Catalysis, 2016, 358, 1916-1923") was dissolved in anhydrous tetrahydrofuran (50 mL), and the reaction solution was cooled to 0 °C, and added with lithium borohydride (450 mg, 20.6 mmol, Sinopharm Chemical Reagent Co., Ltd.). The reaction solution was stirred at room temperature for 18 h. The reaction solution was added with water (80 mL), extracted with ethyl acetate (80 mL×3), and concentrated under reduced pressure, and the resulting residue was purified by silica gel column chromatography with eluent system A to give the title product **1b** (6.0 g, yield: 94.7%). MS m/z (ESI): 305.9 [M+1]

### Step 2

### 1,4-dibromo-6,7-dihydro-5H-cyclopenta[c]pyridine-3-carbaldehyde 1c

Oxalyl chloride (750 mg, 5.91 mmol, Sinopharm Chemical Reagent Co., Ltd.) was dissolved in dichloromethane (20 mL), and the reaction solution was cooled to -78°C., and added dropwise with dimethyl sulfoxide (800 mg, 10.2 mmol, adamas) and stirred for 15 min. The reaction solution was added dropwise with a solution of compound **1b** (1.5 g, 4.9 mmol) in dichloromethane (10 mL) and stirred at -78°C for 1h. The reaction solution was added with triethylamine (1.25 g, 12.4 mmol, Shanghai Sinopharm Chemical Reagent Co., Ltd.), stirred at -78 °C for 30 min, and then stirred at 0 °C for 1.5 h. The reaction solution was added with water (30 mL), extracted with dichloromethane (30 mL×2), dried over anhydrous sodium sulfate, and concentrated under reduced pressure to give the title crude product **1c** (1.5 g) which was used in the next reaction without purification.

MS m/z (ESI): 303.8 [M+1]

### Step 3

### (S, Z)-N-((1,4-dibromo-6,7-dihydro-5H-cyclopenta[c]pyridin-3-yl)methylene)-2-methylpropane-2-sulfinamide 1e

Compound **1c** (5.0 g, 16.4 mmol) and (S)-2-methylpropane-2-sulfinamide **1d** (1.99 g, 16.4 mmol, Bide Pharmatech Ltd.) were dissolved in dichloromethane (50 mL), and the reaction solution was added with cesium carbonate (6.4 g, 19.6 mmol, Bide Pharmatech Ltd.), stirred at room temperature for 2 h, then added with water (50 mL), extracted with dichloromethane (50 mL×3), and concentrated under reduced pressure. The resulting residue was purified by silica gel column chromatography with eluent system A to give the title product **1e** (4.5 g, yield: 67%).

MS m/z (ESI): 406.9 [M+1]

### Step 4

### (S)-N-(1-(1,4-dibromo-6,7-dihydro-5H-cyclopenta[c]pyridin-3-yl)-2-(3,5-difluorophenyl)ethyl)-2-methylpropane-2-sulfinamide 1f

To a suspension of zinc powder (1.3 g, 20.0 mmol, Shanghai Sinopharm Chemical Reagent Co., Ltd.) in anhydrous tetrahydrofuran (15 mL) was added one drop of 1,2-dibromoethane. Under the state of heating at reflux, two drops of trimethylchlorosilane were added, and the reaction solution was vigorously stirred and refluxed for 15 min. The reaction solution was cooled to 0 °C, added with 1-(bromomethyl)-3,5-difluorobenzene (2.1 g, 10.0 mmol, Bide Pharmatech Ltd.), and stirred at room temperature for 4 h. Compound **1e** (2.7 g, 6.6 mmol) was dissolved in anhydrous N,N-dimethylformamide (13 mL), and the reaction solution was added dropwise with the prepared zinc reagent at 0 °C, stirred at room temperature for 16 h, added with water (50 mL), extracted with ethyl acetate (50 mL×3),and concentrated under reduced pressure. The resulting residue was purified by silica gel column chromatography with eluent system A to give the title product **1f** (2.6 g, yield: 75%).

MS m/z (ESI): 534.7 [M+1]

### Step 5

### 1-(1,4-dibromo-6,7-dihydro-5H-cyclopenta[c]pyridin-3-yl)-2-(3,5-difluorophenyl)ethan-1-amine hydrochloride 1g

Compound **1f** (2.6 g, 6.6 mmol) was dissolved in dichloromethane (20 mL), and the reaction solution was added with 4 M hydrogen chloride solution in dioxane (40 mL), stirred at room temperature for 1 h, and concentrated under reduced pressure to give the title product **1g** (2.3 g, yield: 100%).

MS m/z (ESI): 430.8 [M-35]

### Step 6

### Tert-butyl (1-(1,4-dibromo-6,7-dihydro-5H-cyclopenta[c]pyridin-3-yl)-2-(3,5-difluorophenyl)ethyl)carbamate 1h

To a suspension of compound **1g** (2.3 g, 5.3 mmol) in dichloromethane (30 mL) were added triethylamine (3.3 g, 32.7 mmol, Shanghai Hushi Chemical Co., Ltd.) and di-tert-butyl dicarbonate (2.4 g, 11.0 mmol, Accela ChemBio Co., Ltd.). The reaction solution was stirred at room temperature for 3 h, added with water (30 mL), extracted with dichloromethane (30 mL×3), and concentrated under reduced pressure. The resulting residue was purified by silica gel column chromatography with eluent system A to give the title product **1h** (2.1 g, yield: 74%).

MS m/z (ESI): 530.8 [M+1]

### Step 7

### Tert-butyl (1-(4-bromo-1-(3-methyl-3-(methylsulfonyl)but-1-yn-1-yl)-6,7-dihydro-5H-cyclopenta[c]pyridin-3-yl)-2-(3,5-difluorophenyl)ethyl)carbamate 1j

Compound **1h** (500.0 mg, 0.9 mmol) and compound **1i** (207.0 mg, 1.4 mmol, prepared by the method disclosed in the patent application "WO2018035359A1, intermediate im-14 on page 83") were dissolved in N,N-dimethylformamide (8 mL). Bis(triphenylphosphine)palladium dichloride (80.0 mg, 0.1 mmol, Accela ChemBio Co., Ltd.), cuprous iodide (108.0 mg, 0.6 mmol, Shanghai Sinopharm Chemical Reagent Co., Ltd.) and triethylamine (286.0 mg, 2.8 mmol, Shanghai Hushi Chemical Co., Ltd.) were added. The reaction solution was purged with nitrogen three times, stirred at room temperature for 4 h, added with water (20 mL), extracted with ethyl acetate (20 mL×3),and concentrated under reduced pressure. The resulting residue was purified by silica gel column chromatography with eluent system A to give the title product **1j** (500 mg, yield: 89%).

MS m/z (ESI): 596.8 [M+1]

### Step 8

### Tert-butyl (1-4-(3-amino-4-chloro-1-(2,2,2-trifluoroethyl)-1H-indazol-7-yl)-1-(3-methyl-3-(methanesulfonyl)but-1-yn-1-yl)-6,7-dihydro-5H-cyclopenta[c]pyridin-3-yl)-2-(3,5-difluorophenyl)ethyl)carbamate 1l

Compound **1j** (400.0 mg, 0.7 mmol) and compound **1k** (378.0 mg, 1 mmol, prepared by the method disclosed in the patent application "W2018035359A1, intermediate im-12 on page 82") were dissolved in dioxane (12 mL) and water (2 mL). Di-tert-butyl-(4-dimethylaminophenyl) phosphine palladium dichloride (15.0 mg, 0.02 mmol, Accela ChemBio Co., Ltd.) and potassium phosphate (426.0 mg, 2.0 mmol, J&K Chemical) were added. The reaction solution was purged with nitrogen three times, stirred at 90° C. for 16 h, added with water (20 mL), extracted with ethyl acetate (20 mL×3),and concentrated under reduced pressure. The resulting residue was purified by silica gel column chromatography with eluent system A to give the title product **1l** (160.0 mg, yield: 31%).

MS m/z (ESI): 765.8 [M+1]

### Step 9

### ((S)-1-((R)-4-(4-chloro-3-(N-(methylsulfonyl)methanesulfonamide)-1-(2,2,2-trifluoroethyl)-1H-indazol-7-yl)-1-(3-methyl-3-(methylsulfonyl)but-1-yn-1-yl)-6,7-dihydro-SH-cyclopenta[c]pyridin-3-yl)-2-(3,5-difluorophenyl)ethyl)carbamate 1m-1

### Tert-butyl ((R)-1-((S)-4-(4-chloro-3-(N-(methylsulfonyl)methanesulfonamide)-1-(2,2,2-trifluoroethyl)-1H-indazol-7-yl)-1-(3-methyl-3-(methylsulfonyl)but-1-yn-1-yl)-6,7-dihydro-5H-cyclopenta[c]pyridin-3-yl)-2-(3,5-difluorophenyl)ethyl)carbamate 1m-2

Compound **1l** (160.0 mg, 0.2 mmol) and triethylamine (127.0 mg, 1.3 mmol, Shanghai Hushi Chemical Co., Ltd.) were dissolved in dichloromethane (3 mL), and methanesulfonyl chloride (72.0 mg, 0.6 mmol, Shanghai Sinopharm Chemical Reagent Co., Ltd.) was added. The reaction solution was stirred at room temperature for 1 h, added with water (20 mL), extracted with dichloromethane (20 mL×3), and concentrated under reduced pressure. The resulting residue was purified by silica gel column chromatography with eluent system A to give a racemate. The racemate (170 mg, 0.18 mmol) was subjected to chiral preparation (separation conditions: CHIRALPAK OD chiral preparation column, 5.0 cm I.D.×25 cmL, 10 µm; mobile phase: n-hexane/isopropanol=70/30 (V/V), flow rate: 60 mL/min), and the corresponding fractions were collected and concentrated under reduced pressure to give the title products **1m-1** (92 mg, yield: 48%) and **1m-2** (66 mg, yield: 34%).

**Single-Configuration Compound 1m-1:**

MS m/z (ESI): 921.8 [M+1]

Chiral HPLC analysis: retention time: 4.471 min, chiral purity: 100% (column: CHIRALPAK OD 0.46 cm I.D.×25 cmL, 10 µm; mobile phase: n-hexane/ethanol/dichloromethane/diethylamine=60/30/10/0.1 (V/V/V/V)).

**Single-Configuration Compound 1m-2:**

MS m/z (ESI): 921.8 [M+1]

Chiral HPLC analysis: retention time: 6.579 min, chiral purity: 100% (column: CHIRALPAK OD 0.46 cm I.D.×25 cmL, 10 µm; mobile phase: n-hexane/ethanol/dichloromethane/diethylamine=60/30/10/0.1 (V/V/V/V)).

### Step 10

### N-(7-((R)-3-((S)-1-amino-2-(3,5-difluorophenyl)ethyl)-1-(3-methyl-3-(methylsulfonyl)but-1-yn-1-yl)-6,7-dihydro-SH-cyclopenta[c]pyridin-4-yl)-4-chloro-1-(2,2,2-trifluoroethyl)-1H-indazol-3-yl)-N-(methylsulfonyl)methanesulfonamide hydrochloride 1n-1

Compound **1m-1** (92.0 mg, 0.1 mmol) was dissolved in dichloromethane (0.5 mL), and the reaction solution was added with 4 M hydrogen chloride solution in dioxane (3 mL), stirred at room temperature for 1 h, and concentrated under reduced pressure to give the title product **1n-1** (86 mg, yield: 100%).

MS m/z (ESI): 821.8 [M-35]

### Step 11

### N-((S)-1-((R)-4-(4-chloro-3-(methanesulfonamide)-1-(2,2,2-trifluoroethyl)-1H-indazol-7-yl)-1-(3-methyl-3-(methylsulfonyl)but-1-yn-1-yl)-6,7-dihydro-5H-cyclopenta[c]pyridin-3-yl)-2-(3,5-difluorophenyl)ethyl)-2-((3bS,4aR)-5,5-difluoro-3-(trifluoromethyl)-3b,4, 4a,5-tetrahydro-1H-cyclopropa[3,4]cyclopenta[1,2-c]pyrazol-1-yl)acetamide I

Compound **1n-1** (86.0 mg, 0.1 mmol), compound **1o** (29.0 mg, 0.1 mmol, prepared by the method disclosed in the patent application "WO2018035359A1, intermediate im-8a on page 80") and 2-(7-benzotriazole oxide)-N,N,N',N'-tetramethylurea hexafluorophosphate (50.0 mg, 0.13 mmol, Sinopharm Chemical Reagent Co., Ltd.) were dissolved in N,N-dimethylformamide (2 mL), and N,N-diisopropylethylamine (65.0 mg, 0.5 mmol, adamas) was added. The reaction solution was stirred at room temperature for 1 h, added with 2 N sodium hydroxide (0.3 mL), stirred at room temperature for 1 h, then added with water (10 mL), extracted with ethyl acetate (10 mL×3),and concentrated under reduced pressure. The resulting residue was purified by high performance liquid chromatography (Sharpsil-T C18, 150*30 mm, 5 µm, eluent system: H₂O (0.1% trifluoroacetic acid), acetonitrile) to give the title product **I** (33 mg, yield: 30%).

MS m/z (ESI): 1007.5 [M+1]

¹H NMR (400 MHz, CD₃OD) δ 7.20 (d, 1H), 6.76 (t, 1H), 6.49 (d, 1H), 6.26 (d, 2H), 4.92-4.75 (m, 3H), 4.66-4.59 (m, 1H), 4.01-3.95 (m, 1H), 3.24 (s, 3H), 3.23 (s, 3H), 3.17-3.13 (m, 2H), 3.05-3.00 (m, 1H), 2.91-2.86 (m, 1H), 2.82-2.74 (m, 1H), 2.57-2.41 (m, 3H), 2.18-2.15 (m, 1H), 2.07-1.99 (m, 1H), 1.83 (s, 6H), 1.45-1.40 (m, 1H), 1.09 (s, 1H).

### Biological evaluation:

### Test Example 1: Effect of Compounds of formula (I) on In Vitro Polymerization of HIV-1 Capsid Protein

### I. Materials and instruments

### 1. MAb Anti GST-Eu cryptate (Cisbio)

### 2. MAb Anti 6HIS-XI,665 (Cisbio)

### 3. His tag HIV-1 capsid protein (hereinafter referred to as His-CA)

### 4. GST tag HIV-1 capsid protein (hereinafter referred to as GST-CA)

### 5. 384-well unbound surface microplate, white (Corning)

### 6. Microplate reader (BMG)

### II. Procedures

The HIV-1 capsid proteins aggregate spontaneously in high-salt solutions. Capsid protein derived from HIV-1 NL4-3 strains were separately tagged and expressed with 6His tag and GST tag, and purified. His-CA and GST-CA were mixed at high-salt concentrations, and the level of polymerization of capsid proteins was detected using GST antibody labeled with Eu3+-Cryptate (energy donor) and His antibody labeled with XI,665 (energy acceptor). Since the excitation spectrum of Eu3+-Cryptate overlapped with the excitation spectrum of XI,665, when the capsid proteins with these two tags formed a poly complex, the energy donor and the energy acceptor could be drawn to a sufficiently close distance, the energy donor released part of the captured energy under the excitation of an external light source (such as a xenon lamp or a laser), with the emission wavelength being 620 nM, part of the resonance was transferred to the energy acceptor to excite the energy acceptor, with the emission wavelength being 665 nM, and an optical signal was generated, the signal intensity of which was in direct proportion to the degree of polymerization of the tag proteins.

His-CA was diluted to 2 µM in a clean tube with protein diluent (50 mM Tris-HCl, pH 8.0, 50 mM NaCl, 0.4 mM MgClz, 0.05% Triton X-100, 2% glycerol) while MAb Anti 6HIS-XI,665 at a final concentration of 260 nM was added; GST-CA was diluted to 20 nM with protein diluent in another clean tube while MAb Anti GST-Eu cryptate at a final concentration of 3.33 nM was added and incubated on ice for half an hour. Compounds were first formulated into a concentration of 20 mM with DMSO, then diluted to a first concentration of 2 mM with DMSO and serially 5-fold diluted to the 8th concentration. Control wells were set and added with DMSO, and serially-diluted compounds were then 20-fold diluted with protein diluent to 10-fold working concentrations. Preparation of capsid protein polymerization liquid at 2-fold concentration: 50 mM Tris-HCl, pH 8.0, 1 M NaCl, 800 mM KF; to a pre-cooled white round-bottomed 384-well plate were added 4 uL of His-CA-His antibody mixture, 4 uL of GST-CA-GST antibody mixture, 2 uL of the compounds at 10-fold concentration and 10 uL of capsid protein polymerization reaction liquid at 2-fold concentration, the plate was sealed with a light-shielding sealing plate membrane after uniformly mixing, and the reaction plate was placed in a 37 °C incubator for incubation for 2 h. After incubation, the samples were excited with an excitation light at 337 nM, and signal values at 620 nM and 665 nM were collected for detection, wherein Ratio values= (665 nM/620 nM signal)* 10000 was calculated according to the signal values. EC₅₀ values for compounds were calculated using Graphpad Prism software based on each concentration of the compounds and the corresponding Ratio values.

The EC₅₀ values of compound of formula (I) determined by polyinhibition of HIV-1 coat protein are shown in the following table:

| Example | EC₅₀ (nM) | Emax (%) |
|---|---|---|
| The compound of formula (I) | 9 | 100 |

Conclusion: The compound of formula (I) has a significant inhibition effect on the polymerization of HIV-1 capsid proteins.

### Example 2: Preparation of the amorphous form of the choline salt

The compound of formula (I) (1.00 g) was dissolved in isopropanol (15 mL), and choline hydroxide (268 mg, 47% aqueous solution) was added and reacted at room temperature to obtain a yellow solution. The reaction solution was concentrated to dryness. Then 10 mL of dichloromethane was added to dissolve the solution and concentrated, and dried in vacuum to obtain the product (1.1 g, yield: 99.79%). The product was detected as amorphous by X-ray powder diffractometer and the XRPD spectrum is shown in Figure 1.

The results of Nuclear magnetic resonance (HNMR) of the obtained product show that the molar ratio of the compound of formula (I) to the choline in the salt was about 1:1.

¹H NMR (500 MHz, DMSO-*d₆*) δ 9.00 (d, J = 8.4 Hz, 1H), 6.99 (t, J = 9.4 Hz, 1H), 6.94 (d, J = 7.6 Hz, 1H), 6.69 (d, J = 7.6 Hz, 1H), 6.41 (d, J = 6.3 Hz, 2H), 5.36 (s, 1H), 4.89 (d, J = 16.5 Hz, 1H), 4.78 - 4.69 (m, 2H), 4.09 (dd, J = 16.1, 8.7 Hz, 1H), 3.84 (s, 3H), 3.42 - 3.38 (m, 2H), 3.25 (s, 3H), 3.10 (s, 9H), 3.05 (t, J = 7.9 Hz, 2H), 2.93 - 2.80 (m, 2H), 2.77 (s, 3H), 2.67 - 2.53 (m, 3H), 2.40 (ddd, J = 17.3, 8.6, 4.8 Hz, 1H), 2.09 - 2.01 (m, 1H), 1.93 (dt, J = 12.5, 8.3 Hz, 1H), 1.74 (d, J = 5.5 Hz, 6H), 1.39 (q, J = 7.1 Hz, 1H), 0.98 - 0.92 (m, 1H).

### Example 3: Preparation of the amorphous form of the choline salt

The compound of formula (I) in free state was weighed 10mg, and 0.2 mL of methyl tert-butyl ether was added. After they dissolved, 2.8 µl of 44% choline hydroxide aqueous solution was added and pulped at room temperature. 1.4 mL of n-heptane was added and a solid precipitated. The solid was dried in vacuum to obtain the product. The product was detected as amorphous by X-ray powder diffractometer.

### Example 4: Preparation of crystal form A of the choline salt

The amorphous form of the choline salt of the compound of formula (I) (120 mg) was added to 1 mL of a mixed solvent of dioxane, n-hexane, and isopropyl ether (V/V=2:1:1), and dissolved, and stirred at room temperature to precipitate the solid, and dried the solid in vacuum to obtain the product. The product was detected as crystal form A by X-ray powder diffractometer and the XRPD spectrum is shown in Figure 2, and the characteristic peak position thereof is shown in Table 1.

**Table 1**

| **Peak number** | **2θ value [° or degree]** | **d[Å]** | **Relative Strength%** |
|---|---|---|---|
| 1 | 3.414 | 25.86041 | 23.7 |
| 2 | 5.183 | 17.03601 | 28.3 |
| 3 | 7.140 | 12.37140 | 55.7 |
| 4 | 7.587 | 11.64337 | 41.6 |
| 5 | 8.040 | 10.98817 | 100.0 |
| 6 | 8.596 | 10.27820 | 25.4 |
| 7 | 8.835 | 10.00124 | 29.5 |
| 8 | 11.116 | 7.95316 | 22.6 |
| 9 | 11.310 | 7.81716 | 19.6 |
| 10 | 11.606 | 7.61880 | 20.2 |
| 11 | 12.160 | 7.27289 | 10.9 |
| 12 | 12.751 | 6.93711 | 13.1 |
| 13 | 13.083 | 6.76160 | 11.8 |
| 14 | 13.415 | 6.59480 | 7.7 |
| 15 | 14.634 | 6.04817 | 15.1 |
| 16 | 16.259 | 5.44714 | 17.3 |
| 17 | 16.998 | 5.21205 | 23.3 |
| 18 | 18.291 | 4.84650 | 18.4 |
| 19 | 18.845 | 4.70525 | 23.5 |
| 20 | 20.322 | 4.36640 | 50.4 |
| 21 | 21.024 | 4.22221 | 18.6 |
| 22 | 21.726 | 4.08739 | 32.1 |
| 23 | 22.390 | 3.96751 | 12.2 |
| 24 | 23.240 | 3.82437 | 10.8 |
| 25 | 24.680 | 3.60434 | 14.4 |
| 26 | 25.345 | 3.51128 | 5.4 |
| 27 | 27.081 | 3.29002 | 5.4 |
| 28 | 28.337 | 3.14701 | 8.8 |

### Example 5: Preparation of crystal form A of the choline salt

The amorphous form of the choline salt of the compound of formula (I) (60 mg) was added to 0.5 mL of 4-methyl-2-pentanone, and dissolved, and stirred at room temperature to precipitate the solid, and dried the solid in vacuum to obtain the product. The product was detected as crystal form A by X-ray powder diffractometer.

### Example 6: Preparation of crystal form B of the choline salt

The compound of formula (I) (740 mg) and choline hydroxide (198 mg, 47% aqueous solution) were added to isopropanol and stirred at room temperature, and then filtered. The filter cake was dried in vacuum to obtain the product. The product was detected as crystal form B by X-ray powder diffractometer and the XRPD spectrum is shown in Figure 3, and the characteristic peak position thereof is shown in Table 2. The DSC pattern showed that the peak values of the endothermic peak were 65.75 °C and 178.03 °C. The TGA pattern showed a weight loss of 1.06% at 25°C to150°C.

**Table 2**

| **Peak number** | **2θ value [° or degree]** | **d[Å]** | **Relative Strength%** |
|---|---|---|---|
| 1 | 5.363 | 16.46516 | 13.3 |
| 2 | 8.056 | 10.96604 | 35.6 |
| 3 | 10.761 | 8.21506 | 93.2 |
| 4 | 12.289 | 7.19684 | 28.1 |
| 5 | 14.170 | 6.24541 | 44.2 |
| 6 | 14.835 | 5.96680 | 41.7 |
| 7 | 15.197 | 5.82533 | 19.2 |
| 8 | 15.587 | 5.68036 | 12.5 |
| 9 | 16.218 | 5.46082 | 15.7 |
| 10 | 17.676 | 5.01365 | 23.6 |
| 11 | 19.759 | 4.48951 | 8.9 |
| 12 | 20.657 | 4.29635 | 54.2 |
| 13 | 21.279 | 4.17216 | 32.0 |
| 14 | 21.944 | 4.04714 | 28.2 |
| 15 | 22.751 | 3.90549 | 100.0 |
| 16 | 23.836 | 3.73011 | 8.1 |
| 17 | 24.547 | 3.62354 | 12.2 |
| 18 | 25.061 | 3.55040 | 25.1 |
| 19 | 26.392 | 3.37437 | 3.0 |
| 20 | 27.071 | 3.29121 | 5.6 |
| 21 | 27.459 | 3.24555 | 9.7 |
| 22 | 27.938 | 3.19099 | 69.2 |
| 23 | 29.791 | 2.99657 | 27.6 |
| 24 | 31.698 | 2.82058 | 3.1 |
| 25 | 33.089 | 2.70510 | 3.9 |

### Example 7: Preparation of crystal form B of the choline salt

1 g of the compound of formula (I) was dissolved in 10 mL of ethanol, and 0.28 mL of choline hydroxide (44% wt) was added for reaction, and 40 mL of n-heptane was slowly dropped at room temperature to precipitate the solid, and the solid was dried in vacuum to obtain the product. The product was detected as crystal form B by X-ray powder diffractometer.

### Example 8: Preparation of crystal form B of the choline salt

The amorphous form of the choline salt of the compound of formula (I) (60 mg) was weighed, 0.5 mL of a mixed solvent of methanol and isopropyl ether (V/V=1:1) was slowly added, dissolved, and stirred at room temperature to precipitate the solid, and the solid was dried in vacuum to obtain the product. The product was detected as crystal form B by X-ray powder diffractometer.

### Example 9: Preparation of crystal form B of the choline salt

The amorphous form of the choline salt of the compound of formula (I) (60 mg) was weighed, 0.5 mL of isopropanol was slowly added, dissolved, and stirred at room temperature to precipitate the solid, and the solid was dried to obtain the product. The product was detected as crystal form B by X-ray powder diffractometer.

### Example 10: Preparation of crystal form B of the choline salt

The amorphous form of the choline salt of the compound of formula (I) (60 mg) was weighed, 1.5 mL of a mixed solvent of dichloromethane and isopropyl ether (V/V=2:1) was added, and pulped at room temperature to precipitate the solid, and the solid was dried in vacuum to obtain the product. The product was detected as crystal form B by X-ray powder diffractometer.

### Example 11: Preparation of crystal form B of the choline salt

The amorphous form of the choline salt of the compound of formula (I) (120 mg) was added to 1 mL of a mixed solvent of pure water and isopropanol (V/V=10:1), pulped at room temperature to precipitate the solid, and the solid was dried in vacuum to obtain the product. The product was detected as crystal form B by X-ray powder diffractometer.

### Example 12: Preparation of crystal form B of the choline salt

35mg of the crystal form A of the choline salt of the compound of formula (I) was added to 0.5 mL of isopropanol and pulped at room temperature to precipitate the solid, and the solid was dried in vacuum to obtain the product. The product was detected as crystal form B by X-ray powder diffractometer.

### Example 13: Preparation of B-crystal form of the choline salt

The crystal form A of the choline salt of the compound of formula (I) (35 mg) was added to 0.5 mL of a mixed solvent of dichloromethane and isopropyl ether (V/V=1:1), dissolved, and pulped at room temperature to precipitate the solid, and the solid was dried in vacuum to obtain the product. The product was detected as crystal form B by X-ray powder diffractometer.

### Example 14: Preparation of the amorphous form of the sodium salt

10 mg of the compound of formula (I) was added to 0.2 mL ethyl acetate and dissolved. And then 2.6 µl of 4mol/L sodium hydroxide aqueous solution was added and pulped at room temperature 1.2 mL of n-heptane was added, and the solid was precipitated and dried in vacuum to obtain the product. The product was detected as amorphous by X-ray powder diffractometer.

### Example 15: Preparation of crystal form I of the sodium salt

10 mg of the compound of formula (I) was added to 0.1 mL of methyl tert-butyl ether, and 5.45 µl of 2 mol/L sodium hydroxide aqueous solution was added and stirred at elevated temperature in the range of 5°C-50°C to precipitate a solid, and the solid was dried in vacuum to give the product. The content of sodium ion is 2.13% by ion chromatography, and the molar ratio of the compound of formula (I) to sodium ion is 1:1. The product was detected as crystal form B by X-ray powder diffractometer and the XRPD spectrum is shown in Figure 4, and the characteristic peak position thereof is shown in Table 3. The DSC pattern showed that the peak values of the endothermic peak were 131.81 °C and 278.05 °C. The TGA pattern showed a weight loss of 3.81% at 25°C to 185°C.

**Table 3**

| **Peak number** | **2θ value [° or degree]** | **d[Å]** | **Relative Strength%** |
|---|---|---|---|
| 1 | 4.755 | 18.56765 | 12.7 |
| 2 | 5.167 | 17.08842 | 33.6 |
| 3 | 5.869 | 15.04537 | 100.0 |
| 4 | 9.409 | 9.39215 | 8.0 |
| 5 | 10.671 | 8.28387 | 23.4 |
| 6 | 12.035 | 7.34789 | 8.2 |
| 7 | 13.353 | 6.62541 | 6.5 |
| 8 | 16.031 | 5.52414 | 9.3 |
| 9 | 17.850 | 4.96507 | 23.6 |
| 10 | 17.909 | 4.94901 | 19.4 |
| 11 | 18.677 | 4.74716 | 15.1 |
| 12 | 19.388 | 4.57462 | 49.3 |
| 13 | 21.811 | 4.07153 | 19.6 |
| 14 | 22.646 | 3.92332 | 27.4 |
| 15 | 23.483 | 3.78525 | 8.6 |
| 16 | 24.062 | 3.69551 | 3.5 |
| 17 | 24.974 | 3.56264 | 11.4 |
| 18 | 27.604 | 3.22886 | 16.3 |

### Example 16: Preparation of crystal form I of the sodium salt

10 mg of the crystal form III of the sodium salt of the compound of formula (I) was added to 1 mL of methyl tert-butyl ether (MTBE), pulped and then the solid was precipitated. The solid was dried in vacuum to obtain the product. The product was detected as crystal form I by X-ray powder diffractometer.

### Example 17: Preparation of crystal form II of the sodium salt

200 mg of the compound of formula (I) was added to 4.0 mL of methyl tert-butyl ether, and sodium hydroxide solution (19.84 mg sodium hydroxide dissolved in 0.05 mL water) was added and stirred at room temperature. The solid was precipitated and dried in vacuum to obtain the product. The product was detected as crystal form II by X-ray powder diffractometer and the XRPD spectrum is shown in Figure 5, and the characteristic peak position thereof is shown in Table 4. The DSC spectrum showed that the peak heat absorption peaked at 278.1 °C. The TGA pattern showed a weight loss of 0.92% at 25°C to 150°C.

**Table 4**

| **Peak number** | **2θ value [° or degree]** | **d[Å]** | **Relative Strength%** |
|---|---|---|---|
| 1 | 5.293 | 16.68279 | 44.8 |
| 2 | 5.561 | 15.87811 | 34.3 |
| 3 | 6.788 | 13.01048 | 100.0 |
| 4 | 10.864 | 8.13692 | 40.1 |
| 5 | 11.332 | 7.80185 | 11.0 |
| 6 | 13.181 | 6.71129 | 4.4 |
| 7 | 16.027 | 5.52551 | 7.6 |
| 8 | 17.477 | 5.07014 | 16.6 |
| 9 | 17.814 | 4.97515 | 14.8 |
| 10 | 18.966 | 4.67544 | 14.5 |
| 11 | 20.147 | 4.40387 | 0.8 |
| 12 | 20.950 | 4.23693 | 18.5 |
| 13 | 21.344 | 4.15951 | 3.2 |
| 14 | 22.115 | 4.01635 | 11.4 |
| 15 | 22.736 | 3.90803 | 1.1 |
| 16 | 23.189 | 3.83271 | 4.4 |
| 17 | 23.653 | 3.75855 | 17.7 |
| 18 | 24.509 | 3.62915 | 5.7 |
| 19 | 25.979 | 3.42698 | 4.6 |
| 20 | 26.230 | 3.39482 | 3.9 |
| 21 | 27.156 | 3.28114 | 7.4 |
| 22 | 28.430 | 3.13691 | 1.6 |
| 23 | 29.497 | 3.02575 | 1.1 |
| 24 | 31.730 | 2.81778 | 3.2 |
| 25 | 36.369 | 2.46828 | 3.6 |
| 26 | 38.330 | 2.34640 | 2.0 |

### Example 18: Preparation of crystal form II of the sodium salt

The crystal form I of the compound sodium salt of formula (I) was heated to 160 °C and kept at a constant temperature for 1 minute before being lowered to room temperature to obtain a solid product. The product was detected as crystal form II by X-ray powder diffractometer.

### Example 19: Preparation of crystal form II of the sodium salt

100 mg of the compound of formula (I) was added to 1.0 mL of isopropanol, and sodium hydroxide solution (4.36 mg sodium hydroxide dissolved in 0.025 mL water) was added and stirred at room temperature. 2 mL of n-heptane was added for pulping, the solid was precipitated and dried in vacuum to obtain the product. The product was detected as crystal form II by X-ray powder diffractometer.

### Example 20: Preparation of crystal form III of the sodium salt

2 g of the compound of formula (I), 20 mL of isopropanol, and sodium hydroxide solution (83.32 mg of sodium hydroxide dissolved in 0.2 mL of water) were added, stirred at room temperature, and the solid was precipitated, and the solid was dried in vacuum to obtain the product. The product was defined as crystalline type III by X-ray powder diffractometer. The content of sodium ion is 2.12% by ion chromatography, and the molar ratio of the compound of formula (I) to sodium ion is 1:1. The XRPD spectrum is shown in Figure 6, and the characteristic peak position thereof is shown in Table 5. The DSC spectrum showed that the peak heat absorption peaked at 299.93 °C. The TGA pattern showed a weight loss of 0.15% at 25°C to 240°C.

**Table 5**

| **Peak number** | **2θ value [° or degree]** | **d[Å]** | **Relative Strength%** |
|---|---|---|---|
| 1 | 6.352 | 13.90331 | 100.0 |
| 2 | 8.913 | 9.91303 | 41.5 |
| 3 | 12.297 | 7.19205 | 26.4 |
| 4 | 12.850 | 6.88346 | 3.7 |
| 5 | 14.919 | 5.93318 | 5.5 |
| 6 | 16.346 | 5.41830 | 4.3 |
| 7 | 17.000 | 5.21154 | 3.1 |
| 8 | 18.141 | 4.88620 | 17.5 |
| 9 | 18.585 | 4.77050 | 29.9 |
| 10 | 19.373 | 4.57820 | 12.6 |
| 11 | 20.718 | 4.28391 | 11.4 |
| 12 | 21.216 | 4.18437 | 9.4 |
| 13 | 21.938 | 4.04821 | 6.4 |
| 14 | 23.946 | 3.71310 | 3.2 |
| 15 | 26.014 | 3.42252 | 7.3 |
| 16 | 26.968 | 3.30352 | 2.6 |
| 17 | 27.864 | 3.19931 | 5.0 |
| 18 | 33.121 | 2.70254 | 2.5 |

### Example 21: Preparation of crystal form IV of the sodium salt

500 mg of the compound of formula (I) was added with 8.0 mL of ethanol and sodium hydroxide solution (20.83 mg of sodium hydroxide was dissolved in 0.1 mL of water), and the temperature was raised and lowered within the range of 10°C to 40°C to precipitate a solid, and the solid was dried in vacuum to obtain the product. The product was detected as crystal form IV by X-ray powder diffractometer and the XRPD spectrum is shown in Figure 7, and the characteristic peak position thereof is shown in Table 6. The DSC spectrum showed that the peak heat absorption peaked at 298.70 °C. The TGA pattern showed a weight loss of 0.35% at 25°C-205°C.

**Table 6**

| **Peak number** | **2θ value [° or degree]** | **d[Å]** | **Relative Strength%** |
|---|---|---|---|
| 1 | 6.179 | 14.29264 | 100.0 |
| 2 | 8.242 | 10.71918 | 42.1 |
| 3 | 8.914 | 9.91233 | 12.4 |
| 4 | 11.303 | 7.82210 | 3.3 |
| 5 | 12.509 | 7.07042 | 34.0 |
| 6 | 13.557 | 6.52645 | 12.0 |
| 7 | 13.986 | 6.32691 | 13.5 |
| 8 | 16.762 | 5.28504 | 6.2 |
| 9 | 18.158 | 4.88172 | 5.8 |
| 10 | 18.625 | 4.76031 | 20.6 |
| 11 | 19.522 | 4.54359 | 15.0 |
| 12 | 19.727 | 4.49680 | 14.5 |
| 13 | 20.114 | 4.41109 | 10.2 |
| 14 | 21.454 | 4.13854 | 10.7 |
| 15 | 21.746 | 4.08350 | 13.5 |
| 16 | 22.280 | 3.98683 | 8.2 |
| 17 | 23.836 | 3.73011 | 4.4 |
| 18 | 24.515 | 3.62825 | 4.1 |
| 19 | 25.583 | 3.47920 | 2.3 |

### Example 22: Preparation of crystal form V of the sodium salt

2 g of the compound of formula (I), 20 mL of isopropanol, and sodium hydroxide solution (83.32 mg of sodium hydroxide dissolved in 0.2 mL of water) were added, stirred at room temperature, and the solid was precipitated and dried in vacuum to obtain the product. The product was detected as crystal form V by X-ray powder diffractometer and the XRPD spectrum is shown in Figure 8, and the characteristic peak position thereof is shown in Table 7. The DSC pattern showed that the peak values of the endothermic peak were 114.14 °C and 293.92 °C. The TGA pattern showed a weight loss of 5.12% at 25°C to 170°C.

**Table 7**

| **Peak number** | **2θ value [° or degree]** | **d[Å]** | **Relative Strength%** |
|---|---|---|---|
| 1 | 4.506 | 19.59350 | 30.6 |
| 2 | 5.957 | 14.82344 | 100.0 |
| 3 | 7.986 | 11.06174 | 45.6 |
| 4 | 9.016 | 9.80056 | 9.4 |
| 5 | 10.409 | 8.49186 | 7.9 |
| 6 | 11.172 | 7.91325 | 12.3 |
| 7 | 12.219 | 7.23779 | 22.9 |
| 8 | 13.490 | 6.55847 | 32.5 |
| 9 | 16.883 | 5.24716 | 18.8 |
| 10 | 17.462 | 5.07458 | 6.9 |
| 11 | 18.147 | 4.88460 | 30.9 |
| 12 | 19.213 | 4.61580 | 92.3 |
| 13 | 20.115 | 4.41088 | 4.0 |
| 14 | 20.665 | 4.29470 | 3.7 |
| 15 | 21.300 | 4.16806 | 31.7 |
| 16 | 21.629 | 4.10543 | 18.1 |
| 17 | 22.121 | 4.01522 | 3.1 |
| 18 | 22.703 | 3.91353 | 6.4 |
| 19 | 23.434 | 3.79316 | 15.8 |
| 20 | 24.636 | 3.61071 | 16.0 |
| 21 | 25.518 | 3.48787 | 6.0 |
| 22 | 26.323 | 3.38302 | 16.4 |
| 23 | 27.265 | 3.26821 | 7.7 |
| 24 | 27.945 | 3.19028 | 1.6 |
| 25 | 29.012 | 3.07525 | 11.0 |
| 26 | 31.633 | 2.82621 | 2.8 |
| 27 | 34.059 ° | 2.63020 Å | 5.2 |
| 28 | 37.877 ° | 2.37341 Å | 3.7 |

### Example 23: Preparation of crystal form V of the sodium salt

5 mg of crystal form II, crystal form III or crystal form IV of the sodium salt of the compound of formula (I), or the mixture of two or three crystal forms, 1 mL of isopropyl ether is added, and the mixture is pulped at room temperature or 50°C to separate out a solid, and the solid is dried in vacuum to obtain the product. The product was detected as crystal form V by X-ray powder diffractometer.

### Example 24: Preparation of crystal form V of the sodium salt

5 mg of crystal form III of sodium salt of compound of formula (I), 1 mL of isopropanol was added, the solid was precipitated by pulping at room temperature or 50°C, and the solid was dried in vacuum to give the product. The product was detected as crystal form V by X-ray powder diffractometer.

### Example 25: Preparation of the amorphous form of the potassium salt

10 mg of the compound of formula (I) was dissolved by adding 0.1 mL of acetone, then 5.45 µL of 2 mol/L potassium hydroxide aqueous solution was added and stirred, and the solution remained clear and slowly volatilized at room temperature to obtain a solid powder product. The product was detected as amorphous by X-ray powder diffractometer.

### Example 26: Preparation of crystal form a of the potassium salt

10 mg of the compound of formula (I), 0.1 mL of methyl tert-butyl ether, 5.45 µL of 2 mol/L potassium hydroxide aqueous solution were added, the solid was precipitated with stirring, centrifuged, and the solid was dried in vacuo to give the product. The content of potassium ion is 3.42% by ion chromatography, and the molar ratio of the compound of formula (I) to sodium ion is 1:1. The product was detected as crystal form a by X-ray powder diffractometer and the XRPD spectrum is shown in Figure 9, and the characteristic peak position thereof is shown in Table 8.

**Table 8**

| **Peak number** | **2θ value [° or degree]** | **d[Å]** | **Relative Strength%** |
|---|---|---|---|
| 1 | 5.746 | 15.36719 | 39.6 |
| 2 | 8.304 | 10.63848 | 100.0 |
| 3 | 12.253 | 7.21762 | 15.3 |
| 4 | 20.503 | 4.32822 | 18.8 |

### Example 27: Preparation of the amorphous form of the hydrosulfate

10 mg of the compound of formula (I) was added to 1 mL of ethyl acetate, dissolved and cleared, then 5.45 µL of 2 mol/L aqueous sulfuric acid solution was added and stirred, the solution was kept clarified, and volatilized slowly at room temperature to obtain a solid powder product. The product was detected as amorphous by X-ray powder diffractometer.

### Example 28: Preparation of crystal form A of hydrosulfate

10 mg of the compound of formula (I) was added to 0.1 mL of methyl tert-butyl ether, 5.45 µL of 2 mol/L aqueous sulfuric acid solution was added, the solid was precipitated with stirring, centrifuged, and the solid was dried in vacuum to give the product. Through single crystal structure analysis, the molar ratio of the compound shown in formula (I) to bisulfate ion is 1:1. The product was detected as crystal form A by X-ray powder diffractometer and the XRPD spectrum is shown in Figure 10, and the characteristic peak position thereof is shown in Table 9.

**Table 9**

| **Peak number** | **2θ value [° or degree]** | **d[Å]** | **Relative Strength%** |
|---|---|---|---|
| 1 | 7.101 | 12.43861 | 100.0 |
| 2 | 7.982 | 11.06695 | 15.5 |
| 3 | 9.044 | 9.77053 | 21.6 |
| 4 | 9.438 | 9.36284 | 17.2 |
| 5 | 10.247 | 8.62555 | 14.8 |
| 6 | 10.933 | 8.08598 | 23.0 |
| 7 | 12.113 | 7.30054 | 21.8 |
| 8 | 12.742 | 6.94201 | 33.3 |
| 9 | 13.127 | 6.73921 | 10.4 |
| 10 | 13.538 | 6.53535 | 52.0 |
| 11 | 14.259 | 6.20646 | 11.0 |
| 12 | 14.966 | 5.91472 | 32.0 |
| 13 | 15.489 | 5.71635 | 50.2 |
| 14 | 15.870 | 5.57991 | 85.5 |
| 15 | 16.092 | 5.50334 | 76.1 |
| 16 | 16.583 | 5.34139 | 20.0 |
| 17 | 17.187 | 5.15522 | 21.7 |
| 18 | 17.767 | 4.98812 | 20.1 |
| 19 | 18.206 | 4.86881 | 9.9 |
| 20 | 18.959 | 4.67715 | 43.1 |
| 21 | 20.570 | 4.31425 | 57.2 |
| 22 | 21.215 | 4.18459 | 27.7 |
| 23 | 21.603 | 4.11030 | 33.1 |
| 24 | 22.055 | 4.02705 | 40.7 |
| 25 | 23.077 | 3.85102 | 41.0 |
| 26 | 23.649 | 3.75912 | 78.5 |
| 27 | 24.320 | 3.65694 | 58.3 |
| 28 | 25.486 | 3.49223 | 6.2 |
| 29 | 26.423 | 3.37044 | 26.7 |
| 30 | 26.799 | 3.32404 | 30.6 |
| 31 | 27.668 | 3.22156 | 22.7 |
| 32 | 28.280 | 3.15318 | 22.8 |
| 33 | 29.820 | 2.99376 | 22.2 |
| 34 | 31.260 | 2.85907 | 16.8 |
| 35 | 32.119 | 2.78451 | 21.4 |

### Example 29: Preparation of the amorphous form of the calcium salt

10 mg of the compound of formula (I) was added to 1 mL of acetone, after dissolving and clearing 0.81 mg of calcium hydroxide was added and the solution was kept clear and evaporated slowly at room temperature to give a solid powder product. The product was detected as amorphous by X-ray powder diffractometer.

### Example 30: Preparation of the amorphous form of the arginine salt

10 mg of the compound of formula (I) was added to 1 mL of ethanol/water (V/V, 95:5), dissolved clear and then added to 10.91 µL of 1 mol/L arginine salt solution after which the solution remained clear and slowly evaporated at room temperature to give a solid powder product. The product was detected as amorphous by X-ray powder diffractometer.

### Example 31: Preparation of the amorphous form of the lysine salt

10 mg of the compound of formula (I) was added to 1 mL of methyl tert-butyl ether, dissolved clear and then added to 10.91 µL of a 1 mol/L lysine solution after which the solution remained clear and evaporated slowly at room temperature to give a solid powder product. The product was detected as amorphous by X-ray powder diffractometer.

### Example 32: Preparation of the amorphous form of the meglumine salt

10 mg of the compound of formula (I) was added to 1 mL of acetone, dissolved clear and then added to 10.91 µL of 1 mol/L glucosamine solution after which the solution remained clear and evaporated slowly at room temperature to give a solid powder product. The product was detected as amorphous by X-ray powder diffractometer.

### Example 33: Preparation of the amorphous form of the ethanolamine salt

10 mg of the compound of formula (I) was added to 1 mL of ethanol/water (V/V, 95:5), dissolved clear and then added to 6.61 µL of a 1.65 mol/L ethanolamine solution after which the solution remained clear and evaporated slowly at room temperature to give a solid powder product. The product was detected as amorphous by X-ray powder diffractometer.

### Example 34: Preparation of the amorphous form of the hydrochloride

10 mg of the compound of formula (I) was added to 1 mL of ethanol/water (V/V, 95:5), dissolved clear and then added to 5.45 µL of a 2 mol/L hydrochloric acid solution after which the solution remained clear and slowly evaporated at room temperature to give a solid powder product. The product was detected as amorphous by X-ray powder diffractometer.

### Example 35: Preparation of the amorphous form of the tartrate

10 mg of the compound of formula (I) was added to 1 mL of ethanol/water (V/V, 95:5), dissolved clear and then added to 10.91 µL of a 1 mol/L tartaric acid solution after which the solution remained clear and slowly evaporated at room temperature to give a solid powder product. The product was detected as amorphous by X-ray powder diffractometer.

### Example 36: Preparation of the amorphous form of the maleate

10 mg of the compound of formula (I) was added to 1 mL of methyl tert-butyl ether, dissolved clear and then added to 10.91 µL of a 1 mol/L maleic acid solution after the solution remained clear and slowly evaporated at room temperature to give a solid powder product. The product was detected as amorphous by X-ray powder diffractometer.

### Example 37: Preparation of the amorphous form of the citrate

10 mg of the compound of formula (I) was added to 1 mL of methyl tert-butyl ether, dissolved clear and then added to 10.91 µL of a 1 mol/L citric acid solution after the solution remained clear and slowly evaporated at room temperature to give a solid powder product. The product was detected as amorphous by X-ray powder diffractometer.

### Example 38: Preparation of the amorphous form of the malate

10 mg of the compound of formula (I) was added with 1mL of methyl tert-butyl ether, and after dissolving, 10.91µL of 1 mol/L malic acid solution was added, and the solution remained clear and slowly volatilized at room temperature to obtain a solid powder product. The product was detected as amorphous by X-ray powder diffractometer.

### Example 39: Preparation of the amorphous form of the p-toluenesulfonate

10 mg of the compound of formula (I) was added with 1mL of methyl tert-butyl ether, and after dissolving, 10.91µL of 1 mol/L p-toluenesulfonic acid solution was added, and the solution remained clear and slowly volatilized at room temperature to obtain a solid powder product. The product was detected as amorphous by X-ray powder diffractometer.

### Example 40: Preparation of the amorphous form of the mesylate

10 mg of the compound of formula (I) was added with 1mL of methyl tert-butyl ether, and after dissolving, 10.91µL of 1 mol/L methanesulfonic acid solution was added, and the solution remained clear and slowly volatilized at room temperature to obtain a solid powder product. The product was detected as amorphous by X-ray powder diffractometer.

### Example 41: Study on the hygroscopicity of different salt crystal forms of the compound of formula (I)

Using Surface Measurement Systems advantage, at 25°C, the humidity starts from 50%, the humidity range is 0% to 95%, the step is 10%. The judgment standard was that the dM/dT of each gradient mass change is less than 0.002%, TMAX360min, and the cycle was two times. The hygroscopicity of different salt crystal forms of the compound of formula I was investigated respectively. The experimental results are as shown in Table 10:

**Table 10: Hygroscopicity of different salt crystal forms of the compound of formula I**

| Test sample | 0.0%RH-95.0%RH | 20.0%RH-80.0%RH | Crystal form |
|---|---|---|---|
| Crystal form A of hydrosulfate | 4.55% | 1.59% (slightly hygroscopic) | Unchanged |
| Crystal form II of the sodium salt | 34.16% | 11.61% (slightly hygroscopic) | Unchanged |
| Crystal form III of the sodium salt | 2.04% | 0.73% (slightly hygroscopic) | Unchanged |
| Crystal form IV of the sodium salt | 1.03% | 0.45% (slightly hygroscopic) | changed |
| Crystal form a of the potassium salt | 8.77% | 2.86% (slightly hygroscopic) | Unchanged |
| Crystal form B of the choline salt | 9.92% | 5.96% (slightly hygroscopic) | Unchanged |

### Example 42: Study on the stability of factors affecting different salt crystal forms of the compound of formula (I)

The crystal form A of hydrosulfate, the crystal form II of the sodium salt, the crystal form III of the sodium salt, the crystal form a of the potassium salt and the crystal form B of the choline salt of the compound of formula (I) were placed open and flat, and the stability of the samples was investigated under high temperature (40 ° C, 60 ° C) and high humidity (RH 75%, RH 92.5%) conditions. The sampling observation period was 30 days, and the experimental results are respectively shown in Tables 11-15:

**Table 11**

| Conditions | Time (days) | A crystal form of hydrosulfate | | |
|---|---|---|---|---|
| | | Color, properties | Purity % | Crystal form |
| Start | 0 | White solid | 99.09 | A |
| 40°C | 5 | White solid | 99.06 | Unchanged |
| | 10 | White solid | 99.01 | Unchanged |
| | 30 | White solid | 99.03 | Unchanged |
| 60°C | 5 | White solid | 96.46 | Unchanged |
| | 10 | White solid | 96.22 | Unchanged |
| | 30 | White solid | 95.17 | Unchanged |
| 75% RH | 5 | White solid | 99.11 | Unchanged |
| | 10 | White solid | 99.11 | Unchanged |
| | 30 | White solid | 99.05 | Unchanged |
| 92.5% RH | 5 | White solid | 99.18 | Unchanged |
| | 10 | White solid | 99.02 | Unchanged |
| | 30 | White solid | 99.07 | Unchanged |

The results showed that: at 40°C, 60°C, 75% RH and 92.5% RH, the crystal form A of hydrosulfate of the compound of formula I had not transformed and had good physical stability. In addition to high temperature (60°C) conditions, it has good chemical stability under conditions of 40°C, 75% RH, and 92.5% RH.

**Table 12**

| Conditions | Time (days) | II-crystal form of the sodium salt | | |
|---|---|---|---|---|
| | | Color, properties | Purity % | Crystal form |
| Start | 0 | White solid | 97.76 | II |
| 40°C | 5 | White solid | 97.88 | Unchanged |
| | 10 | White solid | 97.83 | Unchanged |
| | 30 | White solid | 97.88 | Unchanged |
| 60°C | 5 | White solid | 97.86 | Unchanged |
| | 10 | White solid | 97.88 | Unchanged |
| | 30 | White solid | 97.90 | Unchanged |
| 75% RH | 5 | White solid | 97.84 | Unchanged |
| | 10 | White solid | 97.84 | Unchanged |
| | 30 | White solid | 97.82 | Unchanged |
| 92.5% RH | 5 | White solid | 97.84 | Unchanged |
| | 10 | White solid | 97.85 | Unchanged |
| | 30 | White solid | 97.82 | Unchanged |

The results showed that: at 40°C, 60°C, 75% RH and 92.5% RH, the crystal form II of the sodium salt of the compound of formula I had not transformed and had good physical and chemical stability.

**Table 13**

| Conditions | Time (days) | Crystal form III of the sodium salt | | |
|---|---|---|---|---|
| | | Color, properties | Purity % | Crystal form |
| Start | 0 | White solid | 98.23 | III |
| 40°C | 5 | White solid | 97.98 | Unchanged |
| | 10 | White solid | 98.10 | Unchanged |
| | 30 | White solid | 98.06 | Unchanged |
| 60°C | 5 | White solid | 98.05 | Unchanged |
| | 10 | White solid | 98.08 | Unchanged |
| | 30 | White solid | 97.99 | Unchanged |
| 75% RH | 5 | White solid | 98.08 | Unchanged |
| | 10 | White solid | 98.10 | Unchanged |
| | 30 | White solid | 98.00 | Unchanged |
| 92.5% RH | 5 | White solid | 97.94 | Unchanged |
| | 10 | White solid | 97.94 | Unchanged |
| | 30 | White solid | 97.89 | Unchanged |

The results showed that: at 40°C, 60°C, 75% RH and 92.5% RH, the crystal form III of the sodium salt of the compound of formula I had not transformed and had good physical and chemical stability.

**Table 14**

| Conditions | Time (days) | a-crystal form of the potassium salt | | |
|---|---|---|---|---|
| | | Color, properties | Purity % | Crystal form |
| Start | 0 | White solid | 98.03 | a |
| 40°C | 5 | White solid | 97.97 | Unchanged |
| | 10 | White solid | 98.04 | Unchanged |
| | 30 | White solid | 97.96 | Unchanged |
| 60°C | 5 | White solid | 98.01 | Unchanged |
| | 10 | White solid | 98.01 | Unchanged |
| | 30 | White solid | 97.82 | Unchanged |
| 75% RH | 5 | White solid | 97.96 | Unchanged |
| | 10 | White solid | 98.04 | Unchanged |
| | 30 | White solid | 98.02 | Unchanged |
| 92.5% RH | 5 | White solid | 97.96 | Unchanged |
| | 10 | White solid | 98.04 | Unchanged |
| | 30 | White solid | 98.02 | Unchanged |

The results showed that: at 40°C, 60°C, 75% RH and 92.5% RH, the crystal form a of the potassium salt of the compound of formula I had not transformed and had good physical and chemical stability.

**Table 15**

| Conditions | Time (days) | B crystal form of the choline salt | | |
|---|---|---|---|---|
| | | Color, properties | Purity % | Crystal form |
| Start | 0 | White solid | 98.34 | B |
| 40°C | 5 | White solid | 98.34 | Unchanged |
| | 10 | White solid | 98.33 | Unchanged |
| | 30 | White solid | 98.35 | Unchanged |
| 60°C | 5 | White solid | 98.39 | Unchanged |
| | 10 | White solid | 98.36 | Unchanged |
| | 30 | White solid | 98.36 | Unchanged |
| 75% RH | 5 | White solid | 98.35 | Unchanged |
| | 10 | White solid | 98.35 | Unchanged |
| | 30 | White solid | 98.36 | Unchanged |
| 92.5% RH | 5 | White solid | 98.45 | Unchanged |
| | 10 | White solid | 98.34 | Unchanged |
| | 30 | White solid | 98.35 | Unchanged |

The results showed that: at 40°C, 60°C, 75% RH and 92.5%, the crystal form B of the choline salt of the compound of formula I had good physical and chemical stability.

### Example 43: Long-term/accelerated stability of different salt crystal forms of the compound of formula I

The crystal form A of hydrosulfate, the crystal form II of the sodium salt, the crystal form III of the sodium salt, the crystal form a of the potassium salt and the crystal form B of the choline salt of the compound of formula (I) were investigated under conditions of 4 °C, 25 °C, 60% RH, and 40 °C, 75% RH, respectively. The experimental results were shown in Tables 16-20.

**Table 16: Long term/accelerated stability of the crystal form A of hydrosulfate**

| | Storage conditions | Purity % | | | | | Crystal form |
|---|---|---|---|---|---|---|---|
| | | Start | 1 month | 2 months | 3 months | 6 months | |
| Samples | 4 °C | 99.09 | 99.09 | 99.08 | 99.08 | 99.09 | A |
| | 25°C, 60%RH | 99.09 | 99.01 | 98.99 | 99.03 | 98.91 | A |
| | 40°C, 75%RH | 99.09 | 98.83 | 98.99 | 98.98 | 98.76 | A |

**Table 17: Long term/accelerated stability of the crystal form II of the sodium salt**

| | Storage conditions | Purity % | | | | | Crystal form |
|---|---|---|---|---|---|---|---|
| | | Start | 1 month | 2 months | 3 months | 6 months | |
| Samples | 4 °C | 97.76 | 97.83 | 97.83 | 97.79 | 97.77 | II |
| | 25°C, 60%RH | 97.76 | 97.82 | 97.82 | 97.86 | 97.79 | II |
| | 40°C, 75%RH | 97.76 | 97.81 | 97.80 | 97.83 | 97.76 | II |

**Table 18: Long term/accelerated stability of the crystal form III of the sodium salt**

| | Storage conditions | Purity % | | | | | Crystal form |
|---|---|---|---|---|---|---|---|
| | | Start | 1 month | 2 months | 3 months | 6 months | |
| Samples | 4 °C | 98.32 | 98.25 | 98.26 | 98.24 | 98.28 | III |
| | 25°C, 60%RH | 98.32 | 98.34 | 98.22 | 98.28 | 98.20 | III |
| | 40°C, 75%RH | 98.32 | 98.25 | 98.22 | 98.27 | 98.23 | III |

**Table 19: Long term/accelerated stability of the crystal form a of the potassium salt**

| | Storage conditions | Purity % | | | | | Crystal form |
|---|---|---|---|---|---|---|---|
| | | Start | 1 month | 2 months | 3 months | 6 months | |
| Samples | 4 °C | 98.03 | 98.02 | 97.99 | 98.04 | 98.01 | a |
| | 25°C, 60%RH | 98.03 | 98.02 | 98.00 | 98.04 | 98.01 | a |
| | 40°C, 75%RH | 98.03 | 98.04 | 98.06 | 98.08 | 97.99 | Deliquesce |

**Table 20: Long term/accelerated stability of the crystal form B of the choline salt**

| | Storage conditions | Purity % | | | | | Crystal form |
|---|---|---|---|---|---|---|---|
| | | Start | 1 month | 2 months | 3 months | 6 months | |
| Samples | 4 °C | 98.34 | 98.38 | 98.31 | 98.28 | 98.30 | B |
| | 25°C, 60%RH | 98.34 | 98.37 | 98.36 | 98.29 | 98.25 | B |
| | 40°C, 75%RH | 98.34 | 98.37 | 98.32 | 98.28 | 98.23 | B |

Long-term/accelerated stability experiments showed that the crystal form A of hydrosulfate, the crystal form II of the sodium salt, the crystal form III of the sodium salt, the crystal form a of the potassium salt and the crystal form B of the choline salt had good physical and chemical stability when placed under long-term accelerated stability conditions for 6 months.

## Claims

1. A pharmaceutically acceptable salt of a compound of formula (I), wherein the pharmaceutically acceptable salt is selected from choline salt, sodium salt, potassium salt, calcium salt, arginine salt, lysine salt, meglumine salt, ethanolamine salt, hydrosulfate, hydrochloride, tartrate, maleate, citrate, malate, p-toluenesulfonate, and mesylate,

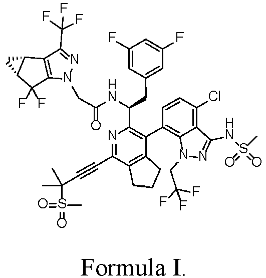

2. The pharmaceutically acceptable salt according to claim 1, wherein, the chemical ratio of the compound of formula (I) to the base molecules or the acid molecules is 1:0.5 to 1:3, preferably 1:0.5, 1:1, 1:2 or 1:3, most preferably 1:1 or 1:2.

3. A method for preparing the pharmaceutically acceptable salt according to claim 1 or 2, comprising the step of forming a salt of a compound of formula (I) with a base or acid.

4. A crystal form A of choline salt of a compound of formula (I), 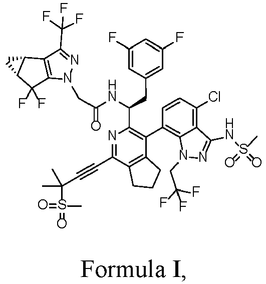 which has an X-ray powder diffraction pattern comprising characteristic peaks at 2θ angles of 5.183, 8.040, 11.116, 16.998, 18.845, 20.322, and 21.726; preferably the X- ray powder diffraction pattern expressed in terms of diffraction angle 2θ angle is shown in FIG. 2.

5. A crystal form B of choline salt of a compound of formula (I), 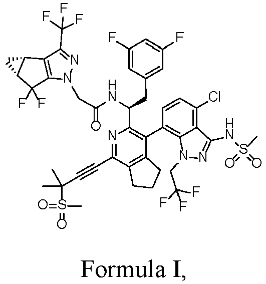 which has an X-ray powder diffraction pattern comprising characteristic peaks at 2θ angles of 8.056, 10.761, 14.170, 14.835, 20.657, 22.751 and 27.938; preferably the X- ray powder diffraction pattern comprising characteristic peaks at 8.056, 10.761, 12.289, 14.170, 14.835, 20.657, 21.279, 21.944, 22.751 and 27.938; more preferably the X- ray powder diffraction pattern comprising characteristic peaks at 8.056, 10.761, 12.289, 14.170, 14.835, 17.676, 20.657, 21.279, 21.944, 22.751, 25.061, 27.938 and 29.791; most preferably the X- ray powder diffraction pattern expressed in terms of diffraction angle 2θ angle is shown in FIG. 3.

6. A crystal form I of sodium salt of a compound of formula (I), 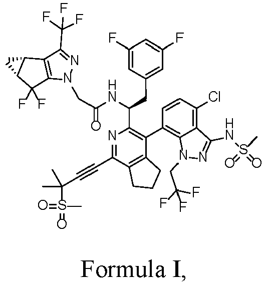 which has an X-ray powder diffraction pattern comprising characteristic peaks at 2θ angles of 5.167, 5.869, 10.671, 17.850, 19.388, 22.646 and 27.604; preferably the X- ray powder diffraction pattern comprising characteristic peaks at 5.167, 5.869, 10.671, 16.031, 17.850, 18.677, 19.388, 22.646, 24.974 and 27.604; more preferably the X- ray powder diffraction pattern comprising characteristic peaks at 5.167, 5.869, 9.409, 10.671, 12.035, 16.031, 17.850, 18.677, 19.388, 21.811, 22.646, 23.483, 24.974 and 27.604; most preferably the X- ray powder diffraction pattern expressed in terms of diffraction angle 2θ angle is shown in FIG. 4.

7. A crystal form II of sodium salt of a compound of formula (I), 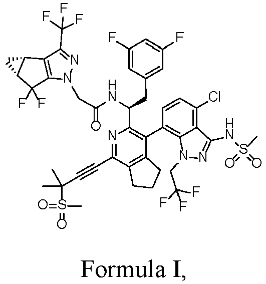 which has an X-ray powder diffraction pattern comprising characteristic peaks at 2θ angles of 5.293, 6.788, 10.864, 17.477, 18.966, 20.950 and 23.653; preferably the X-ray powder diffraction pattern comprising characteristic peaks at 5.293, 6.788, 10.864, 16.027, 17.477, 18.966, 20.950, 22.115, 23.653 and 27.156; more preferably the X- ray powder diffraction pattern comprising characteristic peaks at 5.293, 5.561, 6.788, 10.864, 11.332, 13.181, 16.027, 17.477, 18.966, 20.950, 22.115, 23.653, 24.509, 25.979 and 27.156; most preferably the X- ray powder diffraction pattern expressed in terms of diffraction angle 2θ angle is shown in FIG. 5.

8. A crystal form III of sodium salt of the compound of formula (I), 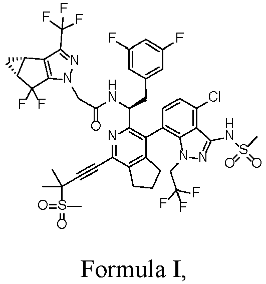 which has an X-ray powder diffraction pattern comprising characteristic peaks at 2θ angles of 6.352, 8.913, 12.297, 18.141, 18.585, 19.373 and 20.718; preferably the X- ray powder diffraction pattern comprising characteristic peaks at 6.352, 8.913, 12.297, 18.141, 18.585, 19.373, 20.718, 21.216, 21.938 and 26.014; more preferably the X- ray powder diffraction pattern comprising characteristic peaks at 6.352, 8.913, 12.297, 14.919, 16.346, 18.141, 18.585, 19.373, 20.718, 21.216, 21.938, 26.014 and 27.864; most preferably the X- ray powder diffraction pattern expressed in terms of diffraction angle 2θ angle is shown in FIG. 6.

9. A crystal form IV of sodium salt of a compound of formula (I), 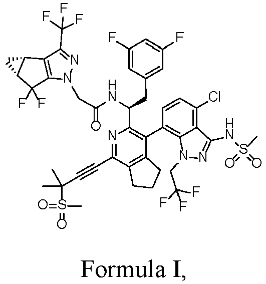 which has an X-ray powder diffraction pattern comprising characteristic peaks at 2θ angles of 6.179, 8.242, 12.509, 13.986, 18.625, 19.522 and 21.746; preferably the X- ray powder diffraction pattern comprising characteristic peaks at 6.179, 8.242, 8.914, 12.509, 13.986, 18.625, 19.522, 21.454, 21.746 and 22.280; more preferably the X- ray powder diffraction pattern comprising characteristic peaks at 6.179, 8.242, 8.914, 12.509, 13.986, 16.762, 18.625, 19.522, 21.454, 21.746, 22.280 and 23.836; most preferably X- ray powder diffraction pattern expressed in terms of diffraction angle 2θ angle is shown in FIG. 7.

10. A crystal form V of the sodium salt of the compound of formula (I), 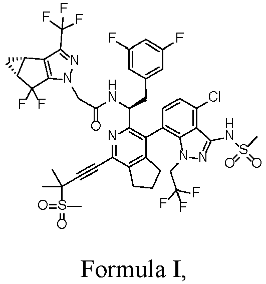 which has an X-ray powder diffraction pattern comprising characteristic peaks at 2θ angles of 4.506, 5.957, 7.986, 13.490, 18.147, 19.213 and 21.300; preferably the X- ray powder diffraction pattern comprising characteristic peaks at 4.506, 5.957, 7.986, 12.219, 13.490, 16.883, 18.147, 19.213, 21.300 and 26.323; more preferably the X- ray powder diffraction pattern comprising characteristic peaks at 4.506, 5.957, 7.986, 11.172, 12.219, 13.490, 16.883, 18.147, 19.213, 21.300, 23.434, 24.636 and 26.323; most preferably the X- ray powder diffraction pattern expressed in terms of diffraction angle 2θ angle is shown in FIG. 8.

11. The crystal form according to any one of claims 4 to 10, wherein, the error range of 2θ angle is ±0.20.

12. A pharmaceutical composition containing the pharmaceutically acceptable salt according of claim 1 or 2, or the crystal form according to any one of claims 4 to 11 and optionally a pharmaceutically acceptable carrier, diluent or excipient.

13. A method for preparing a pharmaceutical composition, comprising the step of mixing the pharmaceutically acceptable salt of claim 1 or 2, or the crystal form according to any one of claims 4 to 11, with a pharmaceutically acceptable carrier, diluent or excipient.

14. A use of the pharmaceutically acceptable salt of claim 1 or 2, the crystal form of any one of claims 4-11, or the composition of claim 12, or the composition prepared by the method according to claim 13 in the preparation of HIV capsid protein inhibitors.

15. A use of the pharmaceutically acceptable salt of claim 1 or 2, the crystal form of any one of claims 4-11, or the composition of claim 12, or the composition prepared by the method of claim 13 in the manufacture of a medicament for the prevention and/or treatment of viral infection diseases, preferably HIV infection.
